# EUROPEAN PATENT APPLICATION

(11) **EP 3 117 851 A1**
(43) Date of publication of application: **18.01.2017**
(21) Application number: 15761571.7
(22) Date of filing: 27.02.2015
(51) Int. Cl.: A61M 5/158, A61J 3/00, A61M 5/162

(54) **INJECTION NEEDLE, INJECTOR, CO-INJECTION DEVICE, AND CO-INJECTION METHOD**

(30) Priority: 12.03.2014 JP 2014048819
(71) Applicant: YUYAMA MFG. CO., LTD., Toyonaka-shi Osaka 561-0841 (JP)
(72) Inventor: KOIKE, Naoki, Toyonaka-shi Osaka 561-0841 (JP); KATAYAMA, Hikaru, Toyonaka-shi Osaka 561-0841 (JP)
(74) Representative: Boco IP Oy Ab
(86) International application number: PCT/JP2015/055819
(87) International publication number: WO 2015/137155

(57) **Abstract**

[Problem] To provide: an injection needle and an injector which are capable of simplifying injection and aspiration processes using an injector and reducing process time; and a co-injection device and co-injection method which perform a co-injection process using the injector.

[Solution] This injection needle (11c) includes a needle tube (211), and a ventilation part (213) that connects, outside the needle tube (211), a first position (first opening (2131)) positioned nearer the rear end (211) side than the tip (2111) of the needle tube (211) and a second position (second opening (2132)) positioned nearer the rear end (2112) side than the first position.

## Description

### TECHNICAL FIELD

The present invention pertains to an injection needle and injector used for suctioning and injecting a liquid, and to a co-injection device and co-injection method for injecting and suctioning the liquid using the injector.

### BACKGROUND ART

Conventionally, co-injection devices for executing a co-injection process of using an injector to suction a liquid drug such as an anticancer agent stored in a medicinal container such as a vial, and to inject the liquid drug into an infusion container are known (for example, see patent document 1). Note that if an anticancer agent or other drug contained in a medicinal container is a powdered medicine, an infusion liquid is suctioned by the injector from the infusion container, and the infusion liquid is injected into the medicinal container to thereby dissolve the powdered medicine, and produce the liquid drug.

### PRIOR ART DOCUMENTS

### Patent Documents

Patent Document 1: Japanese Unexamined Patent Application Publication No. 2012-250016

### SUMMARY OF THE INVENTION

### Problem to be Solved by the Invention

However, with this type of co-injection device, when the infusion liquid is injected into the medicinal container by the injector, suctioning of air from the medicinal container and injection of the infusion liquid into the medicinal container are alternately performed such that excessive positive pressure is not formed in the medicinal container. Moreover, even when a liquid drug is suctioned by the injector from the medicinal container, injection of air into the medicinal container and suctioning of the liquid drug from the medicinal container are alternately executed such that a negative pressure is not excessively formed inside the medicinal container. However, these operations are cumbersome, and lead to a delay in the overall process time.

Therefore, an object of the present invention is to provide an injection needle and injector which are capable of simplifying the injection and suction steps and reducing the process time, and a co-injection device and co-injection method which perform a co-injection process using the injector.

### MEANS FOR SOLVING THE INVENTION

The injection needle according to the present invention is provided with a needle tube; and a ventilation part communicating, outside the needle tube, a first position positioned nearer to a rear end side than a tip end of the needle tube and a second position positioned nearer to the rear end side than the first position.

With the injection needle according to the present invention, the distribution of liquid in the needle tube and the distribution of air in the ventilation part can be performed simultaneously. More specifically, when an injector equipped with the injection needle is used, and liquid is injected into the container with the injection needle puncturing the container to a position between the first position and the second position, air from inside the container is discharged through the ventilation part. In this manner, an injection step of injecting liquid into the container is executed without forming a positive pressure inside the container. Moreover, when an injector equipped with the injection needle is used and liquid is suctioned from the container with the injection needle puncturing the container to a position between the first position and the second position, air flows into the container through the ventilation part. In this manner, a suctioning step of suctioning liquid from the container is executed without forming a negative pressure inside the container. Therefore, according to the present invention, the injection step, the suction step, and the like can be simplified, and the process time can be reduced.

Here, it is conceivable that the ventilation part is a tube-shaped member in which a gap communicating the first position and the second position is formed between the inner circumferential surface of the ventilation part and the outer circumferential surface of the needle tube when the needle tube is inserted into the ventilation part. In this manner, the gap can be used as a ventilation path for distributing air in parallel to the needle tube.

At this time, it is conceivable that an opening communicating the inside and outside of the ventilation part is formed in the ventilation part at one or both of the first position and the second position. Through this, the ventilation path can be embodied with a simple structure.

Moreover, it is also conceivable that an open end part of the gap is positioned at one or both of the first position and the second position of the ventilation part. Through this, the ventilation path can be embodied with a simple structure.

Moreover, it is also conceivable that a first taper part inclined to the inside from the tip end side towards the rear end side is provided on the outer circumferential surface of the needle tube, and a second taper part inclined to the inside from the rear end side towards to the tip end side is provided at one end of the ventilation part. Furthermore, it is also conceivable that in this case, the ventilation part is fixed to the needle tube with an end part of the second taper part abutting the first taper part of the needle tube. In this manner, the abutting location between the first taper part and the second taper part, which is the boundary portion between the needle tube and the ventilation part, is housed inside the needle tube when viewed from the tip end side of the needle tube. Therefore, the concern of coring that can occur when the boundary portion between the needle tube and the ventilation part contacts the rubber stopper of the container when the injection needles punctures the rubber stopper of the container is prevented.

Moreover, it is also conceivable that the ventilation part is provided with a third taper part at one end thereof and the third taper part has a wall thickness increasing towards the outside in a direction from the tip end side to the rear end side. Through this, the injection needle is more easily inserted into the rubber stopper of the container.

Furthermore, it is conceivable that the ventilation part includes a first region circumscribing the outer circumferential surface of the needle tube, and a second region having a gap interposed between the second region and the outer circumferential surface of the needle tube at a position opposing the first region. Here, it is conceivable that an end part of the tip end side of the first region with respect to the third taper part is positioned nearer to the tip end side than the end part of the tip end side of the second region. Moreover, it is conceivable that the inclination angle of the third taper part with respect to the outer circumferential surface of the needle tube becomes smaller in a direction from the first region to the second region in the circumferential direction of the ventilation part. Through this, the pointedness of the tip end of the ventilation part in which a gap is not formed is increased to reduce insertion resistance when the first region punctures the rubber stopper of the container, and when the second region in which the gap is formed punctures the rubber stopper of the container, the pointedness is less than that of the first region, and the occurrence of coring in the second region is suppressed.

It is also conceivable the ventilation part is a groove part formed on the outer circumferential surface of the needle tube across the first position and the second position. Through this, because a groove part may be merely formed on the needle tube, the present invention can be realized with a minimal number of components. Moreover, because the groove part is housed inside the needle tube when viewed from the tip end side of the needle tube, the occurrence of coring originating from the groove part is suppressed. Furthermore, it is conceivable that the ventilation part has a groove part formed on the outer circumferential surface of the needle tube throughout the first position and the second position, and a tube-shaped member which is provided over a part or the whole of the groove part and forms a gap between the inner circumferential surface and the groove part when the needle tube is inserted internally. In this case, the ventilation path is formed by the groove part, and therefore, compared to a structure for which the groove part is not provided, and the ventilation part is a tube-shaped member, the outer diameter of the tube-shaped member can be reduced, and the occurrence of coring is suppressed.

Moreover, it is also conceivable that the ventilation part is a ventilation needle tube fixed to the needle tube in a state of being parallel to the needle tube at a third position nearer to the rear end side than the first position. In this case, when the needle tube and the ventilation needle tube are puncturing the rubber stopper of the container, the insertion ports of the needle tube and the ventilation needle tube for insertion into the rubber stopper are not connected. In this manner, compared to a structure for which the ventilation needle tube is fixed to the outer circumferential surface of the needle tube, damage to the rubber stopper of the container or the occurrence of coring are suppressed.

It is also conceivable that when the injection needle is further provided with a needle base for retaining the rear end side of the needle tube, the first position and the second position are located nearer to the tip end of the needle tube than the needle base. In this manner, the rubber stopper of the container need not to be punctured with the needle base and the occurrence of coring can be more effectively suppressed.

An injector according to the present invention is provided with the injection needle. As described above, with the injector according to the present invention, the injection step, the suction step, and the like can be simplified, and the process time can be reduced.

Moreover, a co-injection device according to the present invention is provided with an injector operation unit capable of operating the injector; and an injection control unit for controlling the injector operation unit and injecting a liquid into a container with the injection needle of the injector puncturing into a rubber stopper of the container as far as a position between the first position and the second position. In this manner, the step of injecting the liquid into the container is executed without forming a positive pressure inside the container, and therefore the injection step can be simplified, and the process time can be reduced.

Moreover, a co-injection device according to the present invention is provided with an injector operation unit capable of operating the injector; and a suction control unit for controlling the injector operation unit and sucking a liquid from inside a container with the injection needle of the injector puncturing into a rubber stopper of the container as far as a position between the first position and the second position. In this manner, the step of suctioning the liquid from the container is executed without forming a negative pressure inside the container, and therefore the suction step can be simplified, and the process time can be reduced.

Furthermore, a co-injection method according to the present invention is a co-injection method executed by a co-injection device, the method including a first puncture step for controlling the operation unit and puncturing the rubber stopper of the container with the injection needle of the injector as far as a position between the first position and the second position; and an injection step for injecting a liquid into the container with the injection needle puncturing the rubber stopper according to the first puncture step. Through this, the step of injecting the liquid into the container can be executed without forming a positive pressure inside the container, and therefore the injection step can be simplified, and the process time can be reduced.

Furthermore, it is conceivable that the co-injection method is further provided with a second puncture step for controlling the operation unit and puncturing the rubber stopper of the container with the injection needle of the injector as far as a position positioned nearer to the tip end side than the first position; and an injection step for sucking the liquid from the container with the injection needle puncturing the rubber stopper according to the second puncture step. Through this, the liquid does not leak from the container via the ventilation part.

Moreover, a co-injection method according to the present invention is a co-injection method executed by the co-injection device, the method thereof including a third puncture step for controlling the operation unit and puncturing the rubber stopper of the container with the injection needle of the injector as far as a position between the first position and the second position; and a suction step for sucking a liquid from the container with the injection needle puncturing the rubber stopper according to the third puncture step. In this manner, the step of suctioning the liquid from the container is executed without forming a negative pressure inside the container, and therefore, the suction step can be simplified, and the process time can be reduced.

### EFFECT OF THE INVENTION

According to the present invention, the injection step, suction step, and the like using an injector can be simplified, and the process time can be reduced.

### BRIEF DESCRIPTION OF THE DRAWINGS

FIG. 1 is a block diagram showing a system configuration of a co-injection device according to an embodiment of the present invention.
FIG. 2 is a perspective view showing an appearance configuration of a co-injection device according to an embodiment of the present invention.
FIG. 3 is a perspective view of a state in which the main door of the co-injection device according to an embodiment of the present invention is opened.
FIG. 4 is an elevation view showing a state with the main door and a portion of the front wall removed from the co-injection device according to an embodiment of the present invention.
FIG. 5 is a perspective view showing a tray used by the co-injection device according to an embodiment of the present invention.
FIG. 6 is a perspective view of the co-injection device according to an embodiment of the present invention as viewed from below.
FIG. 7 is a perspective view showing a holding part of a first robot arm of the co-injection device according to an embodiment of the present invention.
FIG. 8 is a perspective view showing a holding part of a second robot arm of the co-injection device according to an embodiment of the present invention.
FIG. 9 is a plan schematic view showing a tray conveyance unit of the co-injection device according to an embodiment of the present invention.
FIG. 10 is a perspective view showing the mechanism of the tray conveyance unit of the co-injection device according to an embodiment of the present invention.
FIG. 11 is a perspective view showing an ampoule cutter of the co-injection device according to an embodiment of the present invention.
FIG. 12 is a perspective view showing an internal configuration of a stirring device of the co-injection device according to an embodiment of the present invention.
FIG. 13 is a perspective view showing a drug reading unit of the co-injection device according to an embodiment of the present invention.
FIG. 14 is a perspective view showing a needle bend detection unit of the co-injection device according to an embodiment of the present invention.
FIG. 15 is a perspective view showing an internal structure of an injection needle mounting and release device of the co-injection device according to an embodiment of the present invention.
FIG. 16 is a perspective view showing an internal structure of an injection needle mounting and release device of the co-injection device according to an embodiment of the present invention.
FIG. 17 is an image showing an example of an image photographed by a needle insertion confirmation camera of the co-injection device according to an embodiment of the present invention.
FIG. 18 is an image showing an example of the injection needle of the co-injection device according to an embodiment of the present invention.
FIG. 19 is an image showing an example of the injection needle of the co-injection device according to an embodiment of the present invention.
FIG. 20 is an image explaining the injection step of the co-injection device according to an embodiment of the present invention.
FIG. 21 (A) and FIG. 21 (B) are images explaining the suction step of the co-injection device according to an embodiment of the present invention.
FIG. 22 is an image showing an example of an injection needle of the co-injection device according to an embodiment of the present invention.
FIG. 23 is an image showing an example of an injection needle of the co-injection device according to an embodiment of the present invention.
FIG. 24 is an image showing an example of an injection needle of the co-injection device according to an embodiment of the present invention.
FIG. 25 is an image showing an example of an injection needle of the co-injection device according to an embodiment of the present invention.
FIG. 26 is an image showing an example of an injection needle of the co-injection device according to an embodiment of the present invention.
FIG. 27 is an image showing an example of an injection needle of the co-injection device according to an embodiment of the present invention.
FIG. 28 is an image showing an example of an injection needle of the co-injection device according to an embodiment of the present invention.
FIG. 29 is an image showing an example of an injection needle of the co-injection device according to an embodiment of the present invention.
FIG. 30 is an image showing an example of an injection needle of the co-injection device according to an embodiment of the present invention.
FIG. 31 is an image explaining another example of a suction step for the co-injection device according to an embodiment of the present invention.

### MODE FOR CARRYING OUT THE INVENTION

Embodiments of the present invention are explained below with reference to the attached drawings to facilitate an understanding of the present invention. Note that the following embodiments are merely examples that embody the present invention, and are not characteristics that limit the technical scope of the present invention.

### First Embodiment

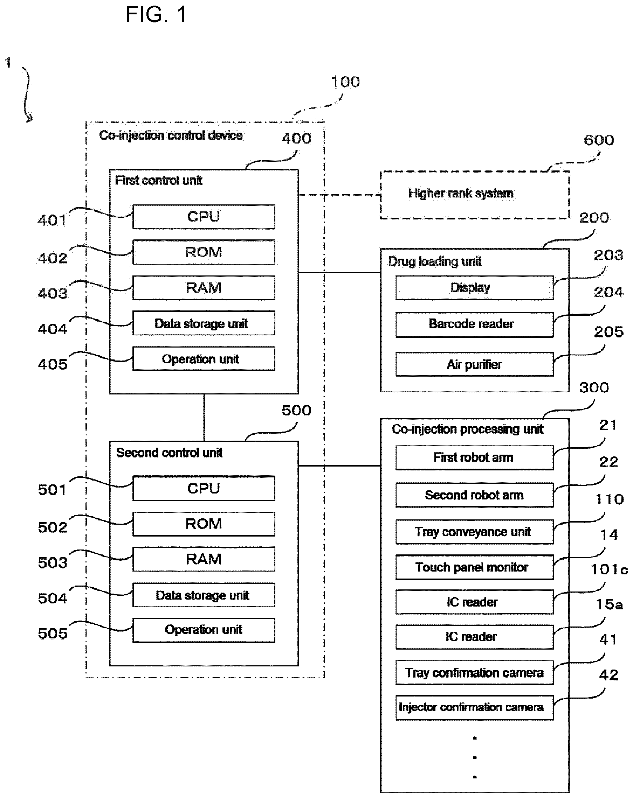

First, a first embodiment of the present invention is described while referencing FIG. 1 to FIG. 21.

### Co-injection Device 1

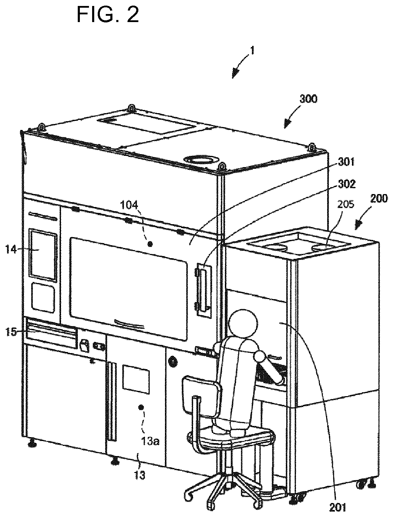

As shown in FIG. 1 and FIG. 2, a co-injection device 1 according to the present embodiment is provided with a co-injection control device 100, a drug loading unit 200, and a co-injection processing unit 300. Furthermore, a co-injection process is executed with the co-injection device 1 to inject an anticancer agent or other drugs shown by preparation data into an infusion liquid container from one or a plurality of medicinal containers, in which a predetermined amount of the drugs is contained, by controlling the operation of the co-injection processing unit 300 using the co-injection control device 100.

### Co-injection Control Device 100

First, a schematic configuration of the co-injection control device 100 is described while referencing FIG. 1. The co-injection control device 100 is provided with a first control unit 400 and a second control unit 500 connected so as to be capable of communication with each other. The first control unit 400 is provided at the drug loading unit 200 side, and the second control unit 500 is provided at the co-injection processing unit 300 side.

Note that the sharing of processing for each of the first control unit 400 and the second control unit 500 explained with the present embodiment is merely one example, and each of the processing procedures of the co-injection process may be executed by either the first control unit 400 or the second control unit 500. Moreover, other embodiments of the co-injection control device 100 are conceivable including embodiments having only one control unit and embodiments having three or more control units. Furthermore, a part or the whole of the processing executed by the first control unit 400 and the second control unit 500 may also be executed by ASIC, DSP, or another type of electronic circuit.

Moreover, the first control unit 400 is capable of communicating with a higher rank system 600 such as an electronic chart system or a prescription preparation control system for inputting preparation data into the co-injection device 1. The preparation data is data for preparation that is generated based on prescription data, or is the prescription data itself. For example, the prescription data includes the prescription issue date, patient ID, patient name, patient date of birth, drug information (drug code, drug name, dosage, and the like), formulation information (internal use, external use, and the like), usage directions (3 times per day after a meal, or the like), medical care type (outpatient, inpatient, or the like), hospital department, hospital ward, hospital room, and the like. Moreover, the preparation data includes patient information, physician information, drug information, drug prescription amount, type of medicinal container (ampoule containing a liquid drug, vial containing a liquid drug, or vial containing a powdered medicine, or the like), detailed preparation information (type and quantity of medicinal containers, injectors, and injection needles used in the co-injection process, and the like), and preparation procedural information (operation details, dissolving liquid, solvent, amount of dissolving liquid, amount of solvent, draw out amount), preparation date, prescription classification, date administered, hospital department, hospital ward, preparation time, and the like.

The first control unit 400 is a personal computer provided with a CPU 401, ROM 402, RAM 403, a data storage unit 404, an operation unit 405, and the like. Various below-described electronic components such as a display 203, a barcode reader 204, and an air purifier 205 which are provided on the drug loading unit 200 are connected to the first control unit 400.

The CPU 401 is a processor for executing processing in accordance with various types of control programs. The ROM 402 is a nonvolatile memory in which a BIOS program or the like that is executed by the CPU 401 is stored in advance. The RAM 403 is a volatile memory or a nonvolatile memory used to deploy various control programs by the CPU 401 and to temporarily store data.

The data storage unit 404 is a hard disk or the like for storing various application programs executed by the CPU 401 and various types of data. More specifically, the preparation data input from the higher rank system 600 is stored in the data storage unit 404.

Here, the first control unit 400 stores the preparation data input from the higher rank system 600, as well as identification information for a below-described tray 110 corresponding to each preparation data. For example, the first control unit 400 associates the preparation data with the tray 110. It is also conceivable that information showing the corresponding relationship between the preparation data and the tray 110 could be input together with the preparation data into the co-injection device 1.

Furthermore, various databases such as a drug master, a patient master, a physician master, a prescription classification master, a hospital department master, and a hospital ward master are stored in the data storage unit 404. For example, the drug master contains drug codes, drug names, JAN codes (or RSS), drug vial codes, classifications (formulation: powdered medicine, tablet medicine, liquid medicine, medicine for external use, and the like), specific gravity, drug types (ordinary drug, anticancer agent, poison, narcotic, powerful medicine, antipsychotic drug, therapeutic drug, and the like), incompatibilities, excipient drugs, precautions, type of medicinal container (ampoule, vial), amount of drug contained per medicinal container unit (predetermined amount), the weight of the medicinal container, and other such information.

Furthermore, a co-injection control program for executing various processing by the CPU 401 is stored in advance in the data storage unit 404. Note that the co-injection control program may be read from a recording medium such as a CD, DVD, BD, or a flash memory by a reading device (not illustrated) provided on the first control unit 400, and installed in the data storage unit 404.

The operation unit 405 includes various types of operation means such as a keyboard, a mouse, or a touch panel for receiving various user operations with respect to the first control unit 400.

The second control unit 500 is a personal computer provided with a CPU 501, ROM 502, RAM 503, a data storage unit 504, an operation unit 505, and the like. Various electronic components such as a robot arm 21, a robot arm 22, a tray conveyance unit 110, a touch panel monitor 14, an IC reader 101 c, an IC reader 15a, a tray confirmation camera 41, and an injector confirmation camera 42, which are described below and provided on the co-injection processing unit 300, are connected to the second control unit 500.

The CPU 501 is a processor for executing various processing in accordance with various control programs. The ROM 502 is a nonvolatile memory in which BIOS or other such programs to be executed by the CPU 501 are stored in advance. The RAM 503 is a volatile memory or a nonvolatile memory used to deploy various control programs by the CPU 501 and to temporarily store data.

The data storage unit 504 is a hard disk or the like for storing various application programs to be executed by the CPU 501 as well as various types of data. More specifically, the co-injection control program for executing the below-described co-injection processing and the like by the CPU 501 is stored in advance in the data storage unit 504. Note that the co-injection control program may be read from a recording medium such as a CD, DVD, BD, or a flash memory by a reading device (not illustrated) provided on the second control unit 500, and installed in the data storage unit 504.

Note also that the present invention may also be perceived as an invention of the co-injection control program for executing various processing by the CPU 401 and the CPU 501, or a recording medium capable of reading from a computer in which the co-injection control program is recorded, with respect to the co-injection control device 100. Moreover, the present invention may also be perceived as an invention of a co-injection method for executing various processing procedures of the co-injection process with respect to the co-injection device 1.

The operation unit 505 includes various types of operation means such as a keyboard, a mouse, or a touch panel for receiving various user operations with respect to the second control unit 500.

### Drug loading unit 200

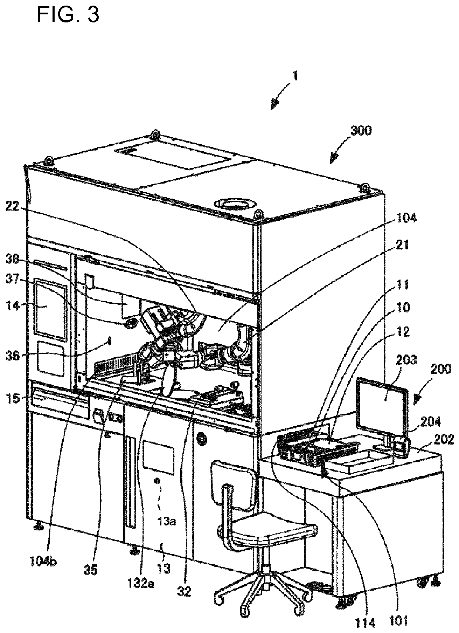

Next, a schematic configuration of the drug loading unit 200 is described while referencing FIG. 2 and FIG. 3.

As shown in FIG. 2 and FIG. 3, the drug loading unit 200 is a clean bench provided with a door 201, an operation table 202, a display 203, a barcode reader 204, and an air purifier 205. Note that as shown in FIG. 3, the drug loading unit 200 and the co-injection processing unit 300 are communicated by a tray insertion slot 114 formed on a side surface of the co-injection processing unit 300.

The display 203 is a display means such as a liquid crystal display or an organic EL display for displaying various types of information in accordance with control instructions from the first control unit 400. More specifically, preparation data or the like that becomes a candidate for the co-injection target of the co-injection device 1 is displayed on the display 203. Moreover, the barcode reader 204 reads a barcode described on a prescription sheet, a preparation instruction sheet, or the like, and inputs the details of the barcode into the first control unit 400. The air purifier 205 supplies air through a prescribed filter to the inside of the drug loading unit 200.

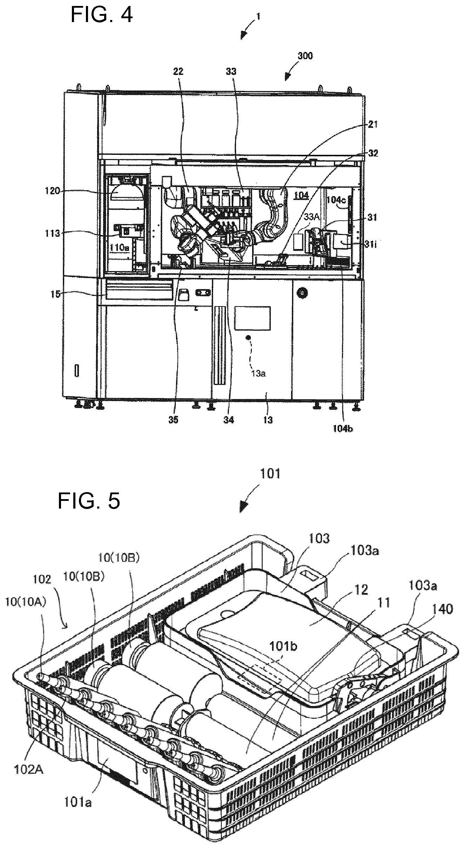

The door 201 is provided on the front surface of the drug loading unit 200, and is capable of opening and closing in the vertical direction. As shown in FIG. 2, the user performs preparation operations for the co-injection process to be executed by the co-injection device 1 with the door 201 slightly open and the user's hands being inserted inside the drug loading unit 200. More specifically, as shown in FIG. 5, a medicinal container 10, an injector 11, an infusion bag 12 (one example of an infusion container), and the like to be used in the co-injection process to be executed by the co-injection device 1 are contained in the tray 101 placed on the operation table 202. The preparation operations include, for example, a loading operation including placing the medicinal container 10, the injector 11, and the infusion bag 12 at prescribed positions in the tray 101, and loading the tray 101 into the co-injection processing unit 300. Below, when the medicinal container 10 is an ampoule, the medicinal container 10 is referred to as an ampoule 10A, and when the medicinal container 10 is a vial, the medicinal container 10 is referred to as a vial 10B.

As shown in FIG. 5, the tray 101 includes electronic paper 101 a for displaying in text form the patient name, treatment, and the like, and an IC tag 101 b (one example of a recording medium) such as an RFID (Radio Frequency Identification) tag capable of reading and writing various information. Identification information for identifying the tray 101 is stored in the IC tag 101 b.

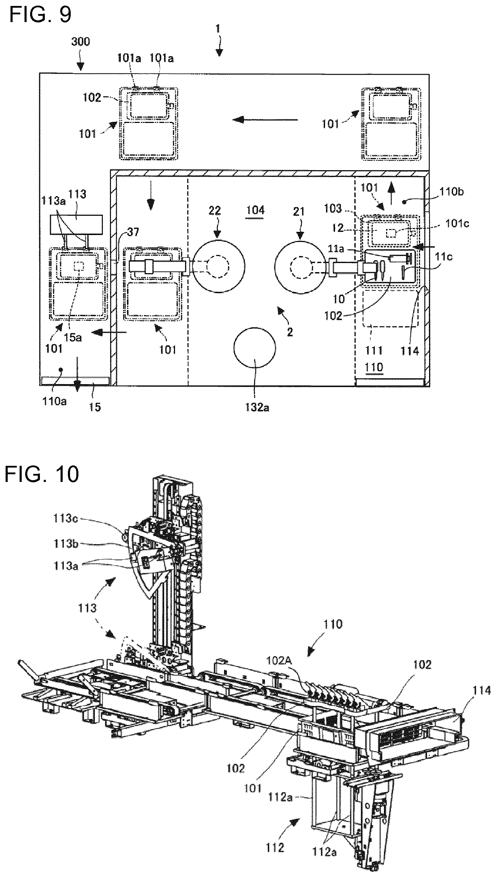

Moreover, the tray 101 has an equipment and material placement section 102 (see FIG. 9) at which the medicinal container 10 and the injector 11 (syringe 11 a, plunger 11 b, injection needle 11c) are placed, and an infusion bag holding part 103 (see FIG. 5) for holding the infusion bag 12. The equipment and material placement section 102 and the infusion bag holding part 103 can be individually placed on and removed from the tray 101.

As shown in FIG. 5, the equipment and material placement section 102 is provided with a support unit 102A for supporting the ampoule 10A in a tilted state. Furthermore, the ampoule 10A is set so as to be standing in a tilted manner at the support unit 102A. In this manner, drugs do not collect in the neck part of the ampoule 10A. Moreover, in addition to the ampoule 10A, the injection needle 11 c of the injector 11, and the like are also set in a state of standing being inclined at the support unit 102A.

The injection needle 11c also includes an injection needle with a syringe filter. More specifically, the injection needle with a syringe filter is used in order to prevent fragments generated when the ampoule 10A is used and the neck of the ampoule 10A is broken, from being injected from the injector 11 into the infusion bag 12, and to prevent the inward flow of the fragments into the injector 11. The syringe filter is a filter that is also generally referred to as a top-shaped filter, and the syringe filter functions to prevent foreign matter other than drugs from passing. For example, a syringe filter available from Pall Corporation Japan is generally known.

On the other hand, as shown in FIG. 5, the vial 10B and the injector 11 are set in a laying down state in the equipment and material placement section 102. Note that at this time, the injector 11 is in a state of the syringe 11 a being separated from the injection needle 11c. The arrangement mode inside the equipment and material placement section 102 described here is of course an example, and the arrangement thereof is not limited to this mode.

Furthermore, as shown in FIG. 5, the infusion bag holding part 103 is provided with a chuck unit 140 for fixing the co-injection port (neck part) of the infusion bag 12. In the preparation operations, the user sets the infusion bag 12 in the infusion bag holding part 103 with the infusion bag 12 being retained by the chuck unit 140. Moreover, the infusion bag holding part 103 is provided with engagement hole parts 103a used when raising and lowering the infusion bag holding part 103.

In addition, after the user sets the medicinal container 10, the injector 11, and the infusion bag 12 in the tray 101, the tray 101 is supplied through the tray insertion slot 114 to the co-injection processing unit 300. Note that it is conceivable that the drug loading unit 200 is also provided with a carry-in mechanism such as a belt conveyor to automatically carry the tray 101 into the co-injection processing unit 300.

### Co-injection Processing Unit 300

Next, a schematic configuration of the co-injection processing unit 300 is described. As shown in FIG. 2 to FIG. 4, the front surface of the co-injection processing unit 300 is provided with a main door 301, an injector removal door 302, a garbage storage chamber door 13, a touch panel monitor 14, a tray discharge port 15, and the like.

The main door 301 opens and closes to access a co-injection process chamber 104 provided on the co-injection processing unit 300 when, for example, cleaning inside the co-injection process chamber 104. Moreover, in the co-injection device 1, in addition to dispensing of the infusion bag 12 into which a drug has been injected, the co-injection device 1 can also dispense the injector 11 filled with a drug. The injector removal door 302 is opened and closed when the injector 11 is retrieved from the co-injection process chamber 104.

The garbage storage chamber door 13 is opened and closed to remove garbage, such as the medicinal container 10 and the injector 11 after use in the co-injection process in the co-injection process chamber 104, from a garbage storage chamber 13a for storing garbage. Moreover, the tray discharge port 15 is opened and closed to retrieve the tray 101 on which the infusion bag 12 is placed after drugs are co-injected by the co-injection process in the co-injection process chamber 104.

The touch panel monitor 14 is a display means such as a liquid crystal display or an organic EL display for displaying various types of information in accordance with control instructions from the second control unit 500. The touch panel monitor 14 can display, for example, an image or video captured by various below-described cameras.

### Co-injection Process Chamber 104

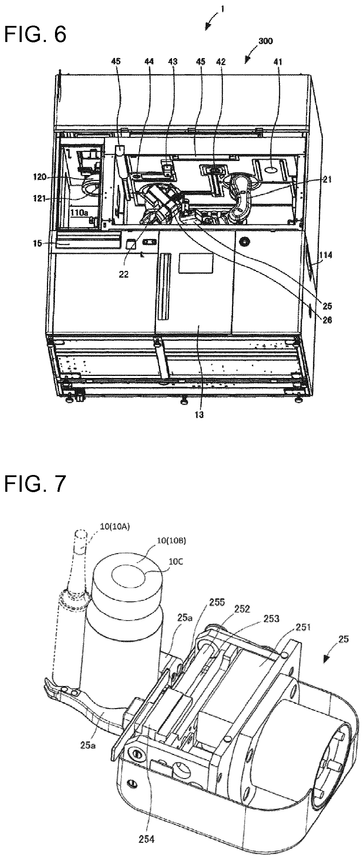

As shown in FIG. 3 and FIG. 4, the co-injection process chamber 104 is provided with a first robot arm 21, a second robot arm 22, an ampoule cutter 31, a stirring device 32, a placement shelf 33, a rotation placement shelf 33A, a drug reading unit 34, a weighing meter 35, a needle bend detection unit 36, a co-injection communication port 37, a transparent window for needle insertion confirmation 38, a garbage lid 132a, and the like. Furthermore, as shown in FIG. 6, the ceiling side of the co-injection process chamber 104 is provided with a tray confirmation camera 41, an injector confirmation camera 42, an injection needle mounting and release device 43, a needle insertion confirmation camera 44, a sterilization lamp 45, and the like.

### First Robot Arm 21, Second Robot Arm 22

The first robot arm 21 and the second robot arm 22 are drive units having multi-joint structures, and are provided in a hanging-down state with base end parts thereof fixed to the ceiling side of the co-injection process chamber 104. The joints of the first robot arm 21 and the second robot arm 22 are respectively configured to have around 5 to 8 axes. Furthermore, in the co-injection device 1, each operation step in the co-injection process is executed by the dual arm type first robot arm 21 and second robot arm 22. More specifically, the second control unit 500 individually drives drive motors provided in each joint of the first robot arm 21 and the second robot arm 22 to execute each operation in the co-injection process by the first robot arm 21 and the second robot arm 22. Note that as long as the co-injection processing unit 300 is of a structure capable of executing the co-injection process, any configuration may be used, including, for example, a configuration having a single robot arm, a configuration having 3 or more robot arms, or a configuration which does not use a robot arm.

As shown in FIG. 6, the first robot arm 21 is provided with a holding part 25 capable of holding equipment and material such as the medicinal container 10 and the injector 11, and the holding part 25 can be moved to an optional position within a predetermined range of movement. The second robot arm 22 is provided with a holding part 26 capable of holding equipment and material such as the medicinal container 10 and the injector 11, and capable of executing drug suction and injection operations by the injector 11. Here, the second robot arm 22 is one example of an injector operation unit. Moreover, the second robot arm 22 is also capable of moving the medicinal container 10, the injector 11, and the like to an optional position within a predetermined range of movement.

As shown in FIG. 7, the holding part 25 of the first robot arm 21 is provided with a pair of gripping claws 25a, a motor 251, two screw shafts 252, 253 rotated by the motor 251, and nut blocks 254, 255 screwed to the screw shafts 252, 253. The pair of gripping claws 25a is respectively fixed to the nut blocks 254, 255. Furthermore, the nut blocks 254, 255 move as a result of rotation of the screw shafts 252, 253, and the pair of gripping claws 25a mutually approaches and separates to hold and release the holding part 25.

Moreover, each of the pair of gripping claws 25a is a gripping part having a recess part suited for retaining the vial 10B, and having, at the tip end side, a recess part suited for retaining the ampoule 10A. FIG 7 shows an aspect in which both the ampoule 10A and the vial 10B are retained, but actually either the ampoule 10A or the vial 10B is retained. Note that a rubber stopper 10C which can be punctured by the injection needle 11 c of the injector 11 is provided at the upper end part of the vial 10B to thereby make the inside of the vial 10B airtight.

Moreover, the holding part 25 is capable of holding an injection needle with a cap or the injector 11 using the pair of gripping claws 25a. Incidentally, the second control unit 500 can measure the diameter of the injector 11 in accordance with the drive amount of the motor 251 when the injector 11 is being held by the pair of gripping claws 25a of the holding part 25. Accordingly, the second control unit 500 can determine whether or not the injector 11 is the injector specified by the preparation details information of the preparation data.

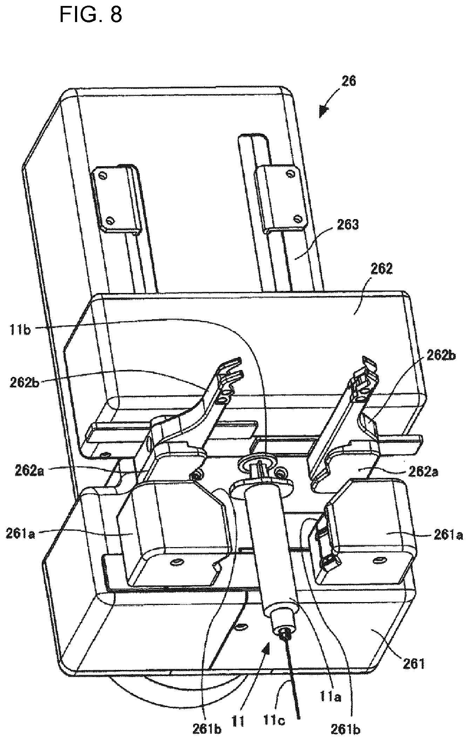

As shown in FIG. 8, the holding part 26 of the second robot arm 22 is provided with an injector holding part 261, a plunger holding part 262, and a moving unit 263. The injector holding part 261 is provided with a pair of gripping claws 261 a for retaining the syringe 11 a of the injector 11. The pair of gripping claws 261 a forms a gripping part in which the gripping claws mutually approach and separate through a mechanism that is the same as the drive mechanism used on the holding part 25, thereby gripping and releasing the syringe 11 a of the injector 11. Moreover, inclined parts 261 b inclining downward from an upper end surface of the gripping claws 261 a towards mutually opposing surfaces are formed in the mutually opposing surfaces of the pair of gripping claws 261 a.

The plunger holding part 262 is provided with a pair of gripping claws 262a for retaining a flange part of the plunger 11 b of the injector 11. The pair of gripping claws 262a forms a gripping part for gripping and releasing the flange part of the plunger 11 b of the injector 11 by mutually approaching and separating through a mechanism that is the same as the drive mechanism used on the holding part 25. Respective gripping claws 262b are fixed to each of the upper surfaces of the gripping claws 262a. The gripping claws 262b form a gripping part in which the gripping claws 262b approach and separate with the approach and separation of the pair of gripping claws 262a, thereby gripping not only the injector 11, but also other equipment and material such as the medicinal container 10. Note that a recess part in which the flange part of the plunger 11 b enters is formed on the upper surface on opposing sides of the pair of gripping claws 262a. Moreover, the tip end of the pair of gripping claws 262b projects further forward than the pair of gripping claws 262a, thereby facilitating gripping of equipment and material such as the ampoule 10A and the vial 10B by the pair of gripping claws 262b. Note that the gripping claws 262b may also be provided on the gripping claws 261 a.

The moving unit 263 can move the plunger holding part 262 in the movement direction of the plunger 11 b of the injector 11. The moving unit 263 moves the plunger 11 b through a drive mechanism which includes, for example, a motor, a screw shaft rotated by the motor, a nut block screwed to the screw shafts, a guide, and the like. The plunger holding part 262 is fixed to the nut block, and moves with the movement of the nut block.

### Tray Conveyance Unit 110

A tray conveyance unit 110 for conveying the tray 101 supplied from the tray insertion slot 114 of a right side end part in FIG. 6 to a tray conveyance end part 110a of a left side end part is provided at the co-injection processing unit 300.

Here, FIG. 9 is a plan schematic view showing an example of a conveyance path of the tray 101 of the tray conveyance unit 110. Note that the inside of the tray conveyance unit 110 is set to a greater positive pressure than the inside of the co-injection process chamber 104. As shown in FIG. 9, the tray conveyance unit 110 is provided such that the tray 101 is conveyed passing the back side of the garbage storage chamber 13a positioned below the garbage lid 132a and below the co-injection process chamber 104. In this manner, the garbage storage chamber 13a can be accessed from the front side of the co-injection device 1. In FIG. 9, the tray 101 moving in the tray conveyance unit 110 is shown by a two-dot chain line in order to show the conveyance path of the tray conveyance unit 110, but this does not mean that a plurality of trays 101 are simultaneously present in the tray conveyance unit 110.

The tray conveyance unit 110 is provided with an IC reader 101c and an IC reader 15a capable of reading information from the IC tag 101 b provided on the infusion bag holding part 10 of the tray 101. For example, the IC reader 101 c and the IC reader 15a may be RFID readers for reading information from an RFID tag. The IC reader 101c is provided at a tray conveyance initiation unit 110b on which the tray 101 is loaded from the tray insertion slot 114, and the IC reader 15a is provided at the tray conveyance end part 110a for discharging the tray 101 from the tray discharge port 15.

Furthermore, when the second control unit 500 determines, based on output from a sensor (not illustrated), that the tray 101 has been inserted into the tray conveyance initiation unit 110b from the tray insertion slot 114, the second control unit 500 reads information from the IC tag 101 b using the IC reader 101 c. Moreover, when the second control unit 500 determines, based on output from the sensor (not illustrated), that the tray 101 has been inserted into the tray conveyance end part 110a, the second control unit 500 reads information from the IC tag 101 b using the IC reader 15a. The second control unit 500 also executes tray comparison processing to determine whether the tray 101 is suitable in accordance with the results read by the IC reader 101c and the IC reader 15a.

Moreover, when the second control unit 500 determines, for example, based on the output of a sensor, that the tray 101 has passed the tray insertion slot 114 and reached a prescribed position inside the tray conveyance unit 110, the second control unit 500 allows a shutter 111 for communication and shielding of the tray conveyance unit 110 and the co-injection process chamber 104 to slide in a horizontal direction. When the shutter 111 opens, the equipment and material placement section 102 is exposed inside the co-injection process chamber 104. FIG. 9 shows a state in which the equipment and material placement section 102 is exposed inside the co-injection process chamber 104.

As shown in FIG. 10, a tray raising and lowering unit 112 is provided on the tray conveyance unit 110 to raise and lower the equipment and material placement section 102 of the tray 101 moved to the inside of the tray conveyance unit 110 through the tray insertion slot 114. The tray raising and lowering unit 12 raises the equipment and material placement section 102 upwardly through driving in the vertical direction of four shafts 112a, which are, for example, provided so as to be capable of raising and lowering.

Furthermore, in the second control unit 500, after the equipment and material placement section 102 has been raised by the tray raising and lowering unit 112, the second control unit 500 captures an image using the tray confirmation camera 41. The tray confirmation camera 41 captures images from above of the medicinal container 10, the injector 11, and the like placed in advance in the predetermined equipment and material placement section 102. The second control unit 500 executes image recognition processing using the image photographed by the tray confirmation camera 41, and determines whether the number of medicinal containers 10 and injectors 11 (syringe 11 a and injection needle 11c), and the like indicated in the preparation data matches the number present in the equipment and material placement section 102.

Moreover, as shown in FIG. 10, a bag raising and lowering unit 113 for raising and lowering the infusion bag holding part 103 is provided at the tray conveyance end part 110a positioned at the left side space of the co-injection process chamber 104. In the second control unit 500, after the tray 101 has been conveyed to the front of the bag raising and lowering unit 113, a hook part 113a of the bag raising and lowering unit 113 is put from below in the engagement hole part 103a. Furthermore, in the second control unit 500, the infusion bag holding part 103 is raised by driving and rotating an arc gear part 113b at which the hook part 113a is formed, using a motor 113c and the co-injection port of the infusion bag 12 is positioned at the co-injection communication port 37. Moreover, in the second control unit 500, controlling the motor 113c enables the bag raising and lowering unit 113 to be driven, the infusion bag holding part 103 to be inclined, and the co-injection port of the infusion bag 12 to be faced upward or downward.

As shown in FIG. 6, a dome type light 120 for illuminating the infusion bag 12 conveyed to the tray conveyance end part 110a and an infusion liquid camera 121 are provided above the tray conveyance end part 110a. The infusion liquid camera 121 is provided at a center part inside the dome type light 120, and reads a barcode attached to the surface of the infusion bag 12. Through this, in the second control unit 500, a decision can be made regarding whether or not the infusion bag 12 is appropriate in accordance with information from the barcode read by the infusion liquid camera 121.

### Ampoule Cutter 31

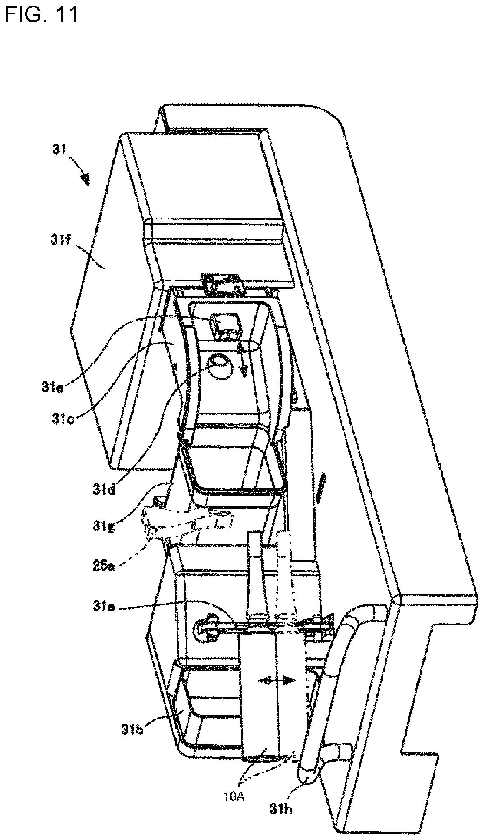

As shown in FIG. 11, the ampoule cutter 31 is provided with a file part 31 a, a waste tray 31 b, a head insertion part 31 c, a drive box 31 f, a waste box 31 g, and a gripping part 31 h.

The file part 31 a is a member for notching of the neck of the ampoule 10A, and waste generated when notching with the file part 31a drops into the waste tray 31b. More specifically, in the co-injection device 1, the first robot arm 21 retains the ampoule 10A, and a notch is provided on the neck of the ampoule 10A by sliding the neck abutting the file part 31 a across the neck of the ampoule 10A.

The head insertion part 31c has a hole 31d into which the head of the ampoule 10A subjected to the notching, is inserted from below, and a pusher 31 e positioned above the hole 31 d and projecting toward the side of the head of the ampoule 10A. Meanwhile, the drive box 31 has a cam provided internally, and a drive motor for driving the cam, and when the cam is driven by the drive motor, the cam causes the pusher 31 e to operate with reciprocating motion in directions of approaching and separating with respect to the head of the ampoule 10A.

Furthermore, in the co-injection device 1, the first robot arm 21 retains the ampoule 10A using the gripping claws 25a, and the head of the ampoule 10A is inserted into the hole 31 d from below such that the upper head part above the neck part is projected upward. Next, when the second control unit 500 drives the drive motor of the drive box 31f so as to move the pusher 31 e in a direction of pushing the head of the ampoule 10A, the head is pushed and broken by the pusher 31 e. At this time, the head broken by the pusher 31 e drops into the waste box 31 g. Note that the gripping part 31 h is used for gripping by the user when the ampoule cutter 31 is slid along a rail 31 i (see FIG. 4) slidably supporting the ampoule cutter 31.

### Stirring Device 32

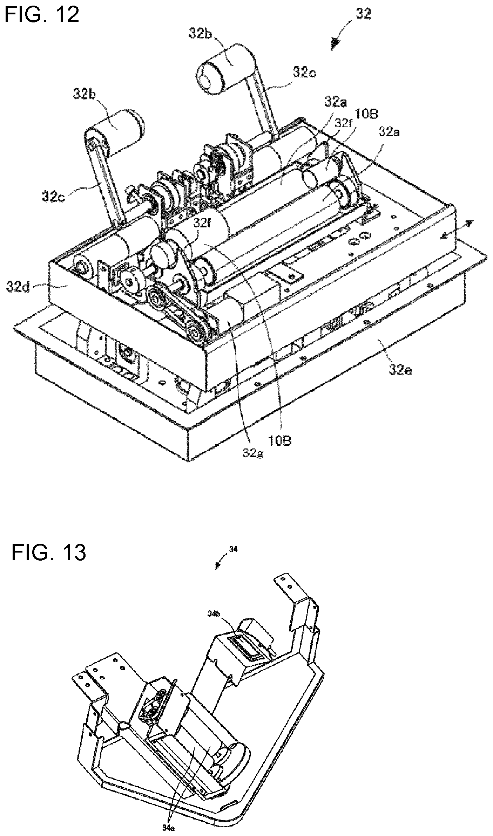

When a drug required to be dissolved such as a powdered medicine (powdered drug) is stored in the vial 10B, an infusion liquid, drug, or the like is injected into the vial 10B to dissolve the drug, and the stirring device 32 is used to produce a mixed drug. More specifically, as shown in FIG. 12, the stirring device 32 is provided with rollers 32a, pressing parts 32b, rotating support units 32c, a support stand 32d, a horizontal oscillating mechanism 32e, support units 32f, a drive motor 32g, and the like.

The two rollers 32a are arranged in an opposing manner separated at a prescribed interval. One of the rollers 32a is supported in a freely rotatable manner, and the other roller 32a is connected to the drive motor 32g. Note that each of the rollers 32a has a long shape in the axial direction, and in the stirring device 32, two vials 10B placed at both ends of the roller 32a in the axial direction can be subjected to stirring simultaneously.

Moreover, the pressing parts 32b are used to press, from above, the vials 10B placed at the rollers 32a, and are driven rollers that rotate in association with the rotation of the medicinal container 10. The rotating support units 32c are used to allow the pressing parts 32b to rotate so as to come into contact with and separate from the medicinal containers 10 by means of a drive motor (not illustrated).

The support stand 32d supports the rollers 32a, the pressing parts 32b, the rotating support units 32c, and the like. The horizontal oscillating mechanism 32e has, for example, a crank mechanism, and can oscillate the support stand 32d in the axial direction of the rollers 32a.

The support units 32f have a U-shaped notch into which the neck of the vial 10B is fitted at both end parts of the rollers 32a in the axial direction. When the vial 10B is placed on the rollers 32a, the neck of the medicinal container 10 engages with the notch. In this manner, when the support stand 32d is oscillated in the axial direction of the rollers 32a by the horizontal oscillating mechanism 32e, the medicinal container 10 follows the oscillation of the rollers 32a in the axial direction thereof and oscillates, and the drugs inside the medicinal container 10 are agitated in the horizontal direction.

Meanwhile, when the vial 10B is placed between the two rollers 32a, and the drive motor 32g is driven, the medicinal container 10 is rotated by the roller 32a connected to the drive motor 32g, and the drugs inside the medicinal container 10 are agitated. Note that at this time, one roller 32a rotates in the same direction as the other roller 32a by the rotation of the medicinal container 10. Moreover, if at least one of the rollers 32a is eccentrically driven, the vial 10B placed on the rollers 32a can also be subjected to agitation in the vertical direction (up and down directions).

### Placement Shelf 33

As shown in FIG. 4, the placement shelf 33 is used for temporarily placing the medicinal container 10, the injector 11, and the like for a co-injection process to be executed in the co-injection device 1. The placement shelf 33 is provided at a position that can be accessed by both the first robot arm 21 and the second robot arm 22. In the placement shelf 33, the vial 10B is placed in a standing state at a predetermined position. Meanwhile, the placement shelf 33 is also provided with an inclined holding part for holding the ampoule 10A in a tilted state, and the ampoule 10A is placed in a tilted state at the inclined holding part. Moreover, a neck retention hole of a predetermined prescribed diameter to which the neck part of the injector 11 fits is formed in the placement shelf 33, and only the syringe of the injector 11 is temporarily placed with the neck part oriented downward without the injection needle 11c attached thereto.

### Rotation Placement Section 33A

While not illustrated, the rotation placement section 33A is used in operations to rotate the injector 11 in the circumferential direction, and is provided at a position that can be accessed by the first robot arm 21. For example, similar to the placement shelf 33, a neck retention hole of a predetermined prescribed diameter to which the neck part of the injector 11 fits is formed on the rotation placement section 33A, and only the syringe of the injector 11 is placed with the neck part oriented downward without the injection needle 11c attached thereto. Furthermore, after the injector 11 is placed on the rotation placement section 33A, the first robot arm 21 can rotate the injector 11 by 180 degrees in the circumferential direction. For example, by repeatedly executing (a) and (b) below, the first robot arm 21 gradually rotates the injector 11 up to 180 degrees in the circumferential direction. (a) The injector 11 is gripped and rotated by a prescribed amount in one direction in the circumferential direction, and then the injector 11 is released, and the angle of the first robot arm 21 is moved by a prescribed amount in the other direction in the circumferential direction. (b) The injector 11 is gripped again, and the injector 11 is rotated by a prescribed amount in one direction in the circumferential direction.

### Drug Reading Unit 34

The drug reading unit 34 reads the barcode indicating information on the contained drug which is described on a label attached to the medicinal container 10 such as the ampoule 10A or the vial 10B. More specifically, as shown in FIG. 13, the drug reading unit 34 is provided with two rollers 34a (one example of a rotation drive means), and a barcode reader 34b (one example of a container reading means). The rollers 34a are arranged in an opposing manner separated by a prescribed interval. One of the rollers 34a is supported so as to be freely rotatable, and the other roller 34a is connected to a drive motor (not illustrated). The two rollers 34a are driven by the drive motor, and thereby a medicinal container 10 placed between the rollers 34a is rotated in the circumferential direction. In this manner, the medicinal container 10 can be subjected to one rotation in the circumferential direction, and therefore the entire area of the label affixed to the medicinal container 10 can be made to face the barcode reader 34b. The barcode reader 34b reads the barcode from the label of the medicinal container 10 rotated by the rollers 34a.

### Weighing Meter 35

The weighing meter 35 is used to measure the weight of the injector 11 in the co-injection process executed with the co-injection device 1, and the measurement results from the weighing meter 35 are input into the second control unit 500. Note that the weighing meter 35 is arranged within the range of movement of the second robot arm 22, and measures the weight of the injector 11 placed by the second robot arm 22.

### Needle Bend Detection Unit 36

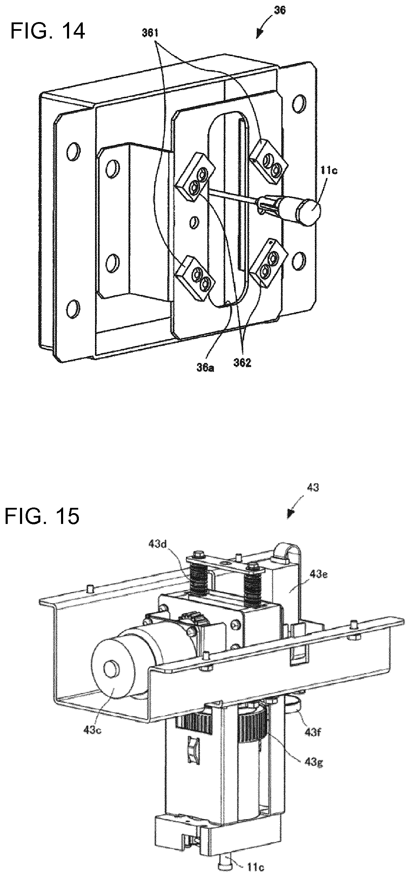

As shown in FIG. 14, an elongated hole 36a, into which the injection needle 11c of the injector 11 can be inserted and moved, is formed in the needle bend detection unit 36. Moreover, the needle bend detection unit 36 is provided with first light sensors 361 and second light sensors 362 arranged with the elongated hole 36a being sandwitched therebetween so as to irradiate and receive light rays such that the mutual light rays are non-parallel. The detection results from the first light sensors 361 and the second light sensors 362 are input into the second control unit 500.

Furthermore, the injection needle 11c mounted to the injector 11 is inserted into the elongated hole 36a, and moved in the up and down directions by means of the second robot arm 22. At this time, when the respective light rays of the first light sensors 361 and the second light sensors 362 are blocked by the injection needle 11c, the first light sensors 361 and the second light sensors 362 turn off. Through this, in the second control unit 500, bending of the injection needle 11c can be detected using position information of the injection needle 11c when the light rays are blocked. Note that other embodiments are also conceivable for detecting needle bend by photographing the injection needle 11c using a camera, and then performing image recognition of the photographed image. Furthermore, if there is bending of the injection needle 11c, in the second control unit 500, the needle tip position, direction, or the like for puncturing of the co-injection port of the infusion bag 12 with the injection needle 11c by the second robot arm 22 is corrected based on the amount of bend of the injection needle 11c.

### Co-injection Communication Port 37

As shown in FIG. 3, the co-injection communication port 37 is formed on a dome-shaped portion projecting to the outside in a side wall of the co-injection process chamber 104, and a notch is formed in the dome-shaped portion for passing the co-injection port of the infusion bag 12 in the vertical direction. Therefore, when the infusion bag holding part 103 is raised, the co-injection port of the infusion bag 12 is positioned inside the co-injection process chamber 104.

### Transparent Window for Needle Insertion Confirmation 38

The transparent window for needle insertion confirmation 38 is a window that enables viewing of the infusion bag 12 at the tray conveyance end part 110a from the co-injection processing unit 300, and is used when photographing an image in order to confirm the state of the injection needle 11 c of the injector 11 inserted into the infusion bag 12.

### Injector Confirmation Camera 42

Moreover, as shown in FIG. 6, the injector confirmation camera 42 is arranged at a ceiling part of the co-injection processing unit 300. In addition, the injector confirmation camera 42 is used when photographing the injector 11 in order to confirm the presence, amount, and the like of a drug suctioned into the injector 11. The injector confirmation camera 42 may be a camera for photographing an image within a predetermined photographing range R1, and by controlling with the second control unit 500, the position and size of the photographing range R1 may be optionally changed. Moreover, as described later, in the co-injection device 1, the injector 11 and the medicinal container 10 are photographed together by the injector confirmation camera 42, and a highly credible inspection image is provided. In the second control unit 500, the image photographed by the injector confirmation camera 42 is recorded in a storage unit such as the data storage unit 4040, the data storage unit 504, or a hard disk provided outside of the co-injection device 1 in order, for example, to use the image to inspect the suitability of the co-injection process to be executed by the co-injection device 1. Furthermore, the second control unit 500 also allows the image photographed by the injector confirmation camera 42 to be displayed on a display device such as the touch panel monitor 14 or the display 203 when an inspection is performed by the user.

### Injection Needle Mounting and Releasing Device 43

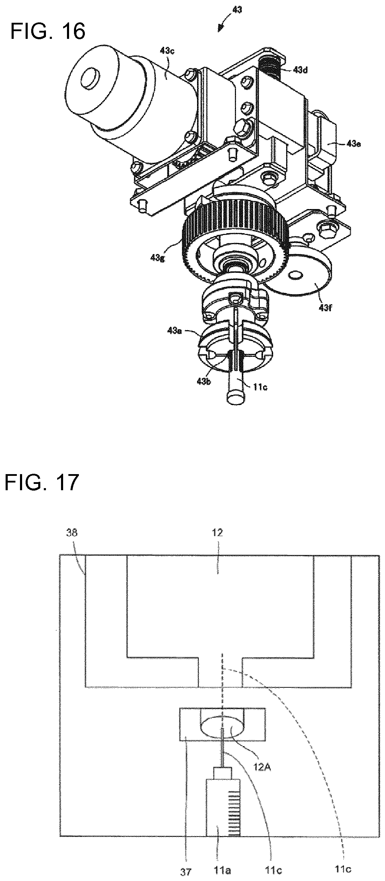

As shown in FIG. 15 and FIG. 16, in the injection needle mounting and releasing device 43, the needle tip of the injection needle 11 c with a cap is inserted upward into a hole part 43b of a chuck unit 43a in which notches are formed. When a motor 43c is driven, the hole part 43b of the chuck unit 43a expands due to a cam mechanism (not illustrated), and the injection needle 11c with a cap can be inserted. When the driving of the motor 43c is stopped, the retained state of the injection needle 11 c with a cap is held by a spring 43d. When a needle turning motor 43e is driven, a gear 43f and a gear 43g are rotated, the chuck unit 43a rotates, and the injection needle 11 c with a cap also rotates. In the injection needle 11c, ribs, which contact when the cap is rotated in the circumferential direction while being mounted to the injection needle 11c, are provided on both the cap and the injection needle 11c. Therefore, when the cap of the injection needle 11 c is rotated with the chuck unit 43a, the injection needle 11c rotates together with the cap and can be mounted to and removed from the syringe 11 b. More specifically, in the injection needle mounting and releasing device 43, replacement of the injection needle 11 c with an injection needle 11c having a syringe filter when using the ampoule 10A can be automatically performed. Moreover, in the injection needle mounting and releasing device 43, the needle tip of the injection needle 11c with a cap is oriented upward and therefore the tip end opening of a syringe main body 11 a from which the injection needle 11 c has been removed is also oriented upward, and dripping of a liquid from the neck part opening of the syringe main body 11 a can be prevented.

### Needle Insertion Confirmation Camera 44

Moreover, the needle insertion confirmation camera 44 photographs the infusion bag 12 positioned outside the co-injection process chamber 104, and the injector 11 inside the co-injection process chamber 104 such that the both are contained within a single image. When the co-injection port of the infusion bag 12 is punctured by the injection needle 11c, in the second control unit 500, the needle insertion confirmation camera 44 photographs an image in the direction of the transparent window for needle insertion confirmation 38. Note that the co-injection port of the infusion bag 12 is provided with a rubber stopper 12A that can be punctured by the injection needle 11 c of the injector 11, and the inside of the infusion bag 12 is in an airtight state.

Furthermore, the image photographed by the needle insertion confirmation camera 44 is displayed, for example, on the touch panel monitor 14. Here, FIG. 17 shows one example of an image photographed by the needle insertion confirmation camera 44. Through this, the user can use the photographed image to confirm whether the tip end side of the injection needle 11 c is positioned inside the infusion bag 12. Note that the photographed image is stored in a storage unit such as a hard disk provided inside or outside the co-injection device 1, for example, for use in a final inspection. Furthermore, when the user operates an OK button on the touch panel monitor 14 on which the photographed image is displayed, and it is thereby determined that the co-injection process has been appropriately completed, the infusion bag 12 is lowered by the bag raising and lowering unit 113, and is returned to the tray 101.

### Sterilization Lamp 45

The sterilization lamp 45, for example, is turned on three hours prior to the startup of the co-injection process. As shown in FIG. 6, one of the two sterilization lamps 45 is provided positioned between the first robot arm 21 and the second robot arm 22. Therefore, the amount of sterilizing light blocked by the first robot arm 21 and the second robot arm 22 is decreased, and the inside of the co-injection process chamber 104 can be thoroughly sterilized. Moreover, an exhaust system is provided in the co-injection processing unit 300 to suction air inside the co-injection process chamber 104 from a slit 104b (see FIG. 3 and FIG. 4) formed on a lower part of a side wall of the co-injection process chamber 104, and to then exhaust the air using an exhaust fan (not illustrated) provided at the upper part of the co-injection process chamber 104. Furthermore, an air supply system is also provided to purify outside air entering from an intake port formed in the ceiling part of the co-injection process chamber 104, and to guide the air to the co-injection process chamber 104 and the like.

### Co-injection Process

Next, an example of procedures for the co-injection process executed by the co-injection processing unit 300 in the co-injection device 1 is described. In the co-injection process, as described below, the second control unit 500 controls the first robot arm 21, the second robot arm 22, and the like, and thereby drugs are suctioned by the injector 11 from one or a plurality of medicinal containers 10 based on the preparation data, and the drugs are injected from the injector 11 into the infusion bag 12.

### Co-injection Process Using the Ampoule 10A

First, the basic operations of the co-injection process when a drug contained in the ampoule 10A is injected into the infusion bag 12 are described. When the tray 101 is supplied to the tray conveyance unit 110, in the second control unit 500, identification information of the tray 101 is read from the IC tag 101 b for the tray 101 using the IC reader 101c. Furthermore, if the identification information of the tray 101 matches the identification information pre-coordinated with the preparation data of the co-injection process, the second control unit 500 opens the shutter 111. Next, the second control unit 500 raises the equipment and material placement section 102 of the tray 101 using the tray raising and lowering unit 112 of the tray conveyance unit 110, and thereby exposes the equipment and material placement section 102 in the co-injection process chamber 104.

Next, in the second control unit 500, the tray confirmation camera 41 photographs the equipment and material placement section 102. Through image recognition processing based on the image photographed by the tray confirmation camera 41, the second control unit 500 determines the position and orientation of the equipment and material such as the ampoule 10A and the injector 11 placed in the equipment and material placement section 102. In particular, in the second control unit 500, each time when the ampoule 10A or the injector 11 is retrieved from the equipment and material placement section 102, the tray confirmation camera 41 photographs the equipment and material placement section 102, and from the photographed image thereof, determines the newest position and orientation of the ampoule 10A and the injector 11.

Next, the second control unit 500 allows the first robot arm 21 to temporarily place, on the placement shelf 33, the injector 11 which is placed on the equipment and material placement section 102 exposed inside the co-injection process chamber 104. Moreover, the second control unit 500 allows the first robot arm 21 to set, at the drug reading unit 34, the ampoule 10 A placed on the equipment and material placement section 102. The second control unit 500 then reads information such as the type of drugs contained in the ampoule 10A with the drug reading unit 34.

The second control unit 500 also allows the first robot arm 21 to set a first injection needle 11c on the injection needle mounting and releasing device 43, and to temporarily place a second injection needle 11c on the placement shelf 33. Here, the first injection needle 11 c is an injection needle without a syringe filter, and the second injection needle 11c is an injection needle with a syringe filter. Note that a cap is attached to the injection needle 11c placed on the equipment and material placement section 102, and the cap is detachable with the injection needle mounting and release device 43.

Furthermore, when all of the equipment and material on the equipment and material placement section 102 is retrieved, the second control unit 500 allows the equipment and material placement section 102 to lower using the tray raising and lowering unit 112 of the tray conveyance unit 110, and return to the tray 101. Note that the second control unit 500 confirms whether or not all of the equipment and material on the equipment and material placement section 102 has been retrieved through image confirmation processing based on the image photographed by the tray confirmation camera 41.

Next, the second control unit 500 closes the shutter 111, and allows the tray conveyance unit 110 to convey the tray 101 to the tray conveyance end part 110a. Next, the second control unit 500 allows the bag raising and lowering unit 113 of the tray conveyance unit 110 to position the co-injection port of the infusion bag 12, which is held by the infusion bag holding part 103 of the tray 101, at the co-injection communication port 37 formed in the co-injection process chamber 104.

Furthermore, the second control unit 500 allows the second robot arm 22 to move the ampoule 10A, set at the drug reading unit 34, to the placement shelf 33. Next, the second control unit 500 retrieves the injector 11 from the placement shelf 33 using the first robot arm 21, and sets it on the second robot arm 22. Next, the second control unit 500 allows the second robot arm 22 to move the injector 11 to the injection needle mounting and release device 43, and set the injection needle 11c on the injector 11. Next, the second control unit 500 allows the second robot arm 22 to move the injector 11 to the needle bend detection unit 36, to detect the presence of a bend on the injection needle 11c.

Subsequently, the second control unit 500 allows the first robot arm 21 to retrieve the ampoules 10A from the placement shelf 33, to break the head part of the ampoule 10A with the ampoule cutter 31. Furthermore, the second control unit 500 allows the first robot arm 21 and the second robot arm 22 to bring the ampoule 10A and the injector 11 into close proximity, and then insert the injection needle 11 c of the injector 11 into the ampoule 10A. Next, the second control unit 500 operates the plunger 11 b using the second robot arm 22 to suction a predetermined amount of drugs with the injector 11 from the ampoule 10A in accordance with the preparation data.

At this time, the first robot arm 21 and the second robot arm 22 gradually tilt the posture of the ampoule 10A and the injector 11. For example, with the mouth part of the ampoule 10A oriented in a vertically upward direction, and the injection needle 11 c of the injector 11 oriented in a vertically downward direction, a certain amount of a drug is suctioned up from the ampoule 10A, and thereafter the ampoule 10A is inclined about 10 degrees with respect to the vertical direction, to form a state of the drug having been moved to the mouth part side (neck part). In this manner, the drug can be suctioned up as much as possible such that no drug material is left behind without letting the tip end of the injection needle 11 c of the injector 11 touch the bottom of the ampoule 10A.

Next, the second control unit 500 controls one or the both of the first robot arm 21 and the second robot arm 22 to move the ampoule 10A from which the drug has been suctioned and the injector 11 which has suctioned the drug, up to a position within the photographing range R1 of the injector confirmation camera 42. Here, when executing the movement processing, the second control unit 500 is one example of a movement control means. Furthermore, the second control unit 500 photographs the ampoule 10A and the injector 11 together using the injector confirmation camera 42, and records the photographed image as an inspection image in the data storage unit 504. Here, the injector confirmation camera 42 is one example of a photographing means when suctioning. For example, the injector confirmation camera 42 photographs the predetermined photographing range R1. On the other hand, it is also conceivable that the second control unit 500 can change the photographing range R1 of the injector confirmation camera 42 such that the ampoule 10A and the injector 11 can be photographed together after being moved by the first robot arm 21 and the second robot arm 22.

Next, the second control unit 500 replaces the injection needle 11c of the injector 11 using the first robot arm 21 and the second robot arm 22. More specifically, the second robot arm 22 moves the injector 11 to the injection needle mounting and release device 43, and mounts the cap to the injection needle 11c. Furthermore, the second control unit 500 rotates the cap using the injection needle mounting and release device 43, and removes the injection needle 11 c from the injector 11. Note that the removal of the injection needle 11c may also be performed by a cap rotation operation by the first robot arm 21 and the second robot arm 22.

Moreover, the second control unit 500 opens the garbage lid 132a, and drops and discards the injection needle 11c gripped by the injection needle mounting and release device 43 using the first robot arm 21 into the garbage storage chamber 13a. Next, the second control unit 500 allows the first robot arm 21 to move the injection needle 11 c with a syringe filter from the placement shelf 33 and to set the injection needle 11c on the injection needle mounting and release device 43.

Next, the second control unit 500 allows the second robot arm 22 to move the injector 11 to the injection needle mounting and release device 43, and to mount the injection needle 11 c into the injector 11. In this case as well, the second control unit 500 moves the injector 11 to the needle bend detection unit 36 using the second robot arm 22, and detects the presence of a bend in the injection needle 11c. In this manner, in the co-injection device 1, when a drug is suctioned from the ampoule 10A, and also when an infusion liquid is injected into the infusion bag 12, the injection needle 11c is replaced, and mixing of fragments from the ampoule 10A into the infusion bag 12 can be prevented.

Furthermore, the second control unit 500 allows the second robot arm 22 to puncture, with the injection needle 11c of the injector 11, the co-injection port of the infusion bag 12 conveyed to the tray conveyance end part 110a, and a mixed drug in the injector 11 is injected into the infusion bag 12. Meanwhile, the second control unit 500 opens the garbage lid 132a, and allows the first robot arm 21 to drop and discard the ampoule 10A into the garbage storage chamber 13a. Moreover, the second control unit 500 allows second robot arm 22 to move the injector 11 to the injection needle mounting and release device 43 and mount the cap to the injection needle 11 c of the injector 11, and thereafter, the injector 11 is dropped and discarded in the garbage storage chamber 13a.

Next, the second control unit 500 reads various types of images photographed by the injector confirmation camera 42 and the like from the data storage unit 504, and displays the images on the touch panel monitor 14. As a result, the user can inspect the suitability of the co-injection process while viewing the touch panel monitor 14.

### Co-injection Process Using the Vial 10B

Next, the basic operations of the co-injection process when a drug is mixed with an infusion liquid and then injected into the infusion bag 12 are described for a case in which the drug contained in the vial 10B is a drug such as a powdered medicine, which must be dissolved.

When the tray 101 is supplied to the tray conveyance unit 110, the second control unit 500 reads the identification information of the tray 101 from the IC tag 101 b of the tray 101 using the IC reader 101c. If the identification information of the tray 101 matches the identification information coordinated in advance to the preparation data of the co-injection process, the second control unit 500 opens the shutter 111. Next, the second control unit 500 raises the equipment and material placement section 102 of the tray 101 using the tray raising and lowering unit 112 of the tray conveyance unit 110, and exposes the equipment and material placement section 102 in the co-injection process chamber 104.

Next, the second control unit 500 photographs the equipment and material placement section 102 using the tray confirmation camera 41. Furthermore, through image confirmation processing based on the image photographed by the tray confirmation camera 41, the second control unit 500 identifies the position and orientation of equipment and material such as the vial 10B and the injector 11 placed on the equipment and material placement section 102. In particular, each time when the vial 10B or the injector 11 is retrieved from the equipment and material placement section 102, the second control unit 500 photographs the equipment and material placement section 102 with the tray confirmation camera 41, and identifies the newest position and orientation of the vial 10B and the injector 11 from the photographed image thereof.

Next, the second control unit 500 allows the first robot arm 21 to temporarily place, on the placement shelf 33, the injector 11 placed in the equipment and material placement section 102 exposed inside the co-injection process chamber 104. Moreover, the second control unit 500 allows the first robot arm 21 to set the vial 10B placed in the equipment and material placement section 102 at the drug reading unit 34. The second control unit 500 then allows the drug reading unit 34 to read information such as the type of drug contained in the vial 10B.

Furthermore, when all of the equipment and material on the equipment and material placement section 102 has been retrieved, the second control unit 500 allows the tray raising and lowering unit 112 of the tray conveyance unit 110 to lower the equipment and material placement section 102 and return it to the tray 101. Note that the second control unit 500 confirms whether or not all of the equipment and material on the equipment and material placement section 102 has been retrieved through image confirmation processing based on the image photographed by the tray confirmation camera 41.

Next, the second control unit 500 closes the shutter 111, and conveys the tray to the tray conveyance end part 110a using the tray conveyance unit 110. The second control unit 500 then uses the bag raising and lowering unit 113 of the tray conveyance unit 110 to position the co-injection port of the infusion bag 12 retained by the infusion bag holding part 103 of the tray 101 at the co-injection communication port 37 formed in the co-injection process chamber 104.

Furthermore, the second control unit 500 allows the second robot arm 22 to move the vial 10B set at the drug reading unit 34 to the placement shelf 33. Meanwhile, in parallel with this movement process, the second control unit 500 also allows the first robot arm 21 to set the injection needle 11c of the injector 11 placed at the equipment and material placement section 102 into the injection needle mounting and release device 43.

Next, the second control unit 500 retrieves the injector 11 from the placement shelf 33 using the first robot arm 21, and sets it on the second robot arm 22. Subsequently, the second control unit 500 allows the second robot arm 22 to move the injector 11 to the injection needle mounting and release device 43, and set the injection needle 11 c in the injector 11. Note that it is also conceivable that the injection needle 11c could have been mounted to the injector 11 when placed on the tray 101. Next, the second control unit 500 allows the second robot arm 22 to move the injector 11 to the needle bend detection unit 36, to detect the presence of any bending of the injection needle 11c.

Subsequently, the second control unit 500 allows the second robot arm 22 to puncture the co-injection port of the infusion bag 12 conveyed to the tray conveyance end part 110a with the injection needle 11 c of the injector 11, and then suction from the infusion bag 12, a dissolved infusion liquid in an amount shown in the preparation data. Meanwhile, the second control unit 500 allows the first robot arm 21 to retrieve the vial 10B placed on the placement shelf 33.

Furthermore, the second control unit 500 allows the first robot arm 21 and the second robot arm 22 to bring the vial 10B and the injector 11 into close proximity respectively, to puncture the vial 10B with the injection needle 11c of the injector 11. Next, the second control unit 500 operates the plunger 11 b using the second robot arm 22, and thereby injects the infusion liquid in the injector 11 into the vial 10B. As a result, the drug in the vial 10B is dissolved by the infusion liquid. At this time, the orientation of injector 11 and the vial 10B is such that the injection needle 11c of the injector 11 faces the vertically downward direction, and the mouth part of the vial 10B faces the vertically upward direction.

Next, the second control unit 500 set the vial 10B, into which the infusion liquid has been injected, using the first robot arm 21, on the stirring device 32. The stirring device 32 then stirs the drugs and infusion liquid inside the vial 10B. When the stirring with the stirring device 32 is completed, the second control unit 500 retrieves the vial 10B from the stirring device 32 using the first robot arm 21.

The second control unit 500 then brings the vial 10B and the injector 11 into close proximity respectively, using the first robot arm 21 and the second robot arm 22, and then punctures the vial 10B with the injection needle 11 c of the injector 11. Next, the second control unit 500 operate the plunger 11 b, using the second robot arm 22 to and thereby suctions the mixed drug inside the vial 10B with the injector 11. At this time, the orientation of the injector 11 and the vial 10B is such that the injection needle 11 c of the injector 11 faces the vertically downward direction, and the mouth part of the vial 10B faces the vertically upward direction. Note that the orientation of the injector 11 and the vial 10B may also be such that the mouth part of the vial 10B faces the vertically downward direction, and the injection needle 11 c of the injector 11 faces the vertically upward direction.

Next, the second control unit 500 controls one or the both of the first robot arm 21 and the second robot arm 22 to move the vial 10B, from which the drug has been suctioned and the injector 11 which has suctioned the drug, up to a position within the photographing range R1 of the injector confirmation camera 42. Here, when executing the movement processing, the second control unit 500 is one example of a movement control means. Furthermore, the second control unit 500 photographs the vial 10B and the injector 11 together using the injector confirmation camera 42, and records the photographed image as an inspection image in the data storage unit 504. For example, the injector confirmation camera 42 photographs the predetermined photographing range R1. On the other hand, it is also conceivable that the second control unit 500 can change the photographing range R1 of the injector confirmation camera 42 such that the vial 10B and the injector 11 can be photographed together after being moved by the first robot arm 21 and the second robot arm 22.

The second control unit 500 then allows the second robot arm 22 to puncture the co-injection port of the infusion bag 12 conveyed to the tray conveyance end part 110a with the injection needle 11c of the injector 11, to thereby inject the mixed drug inside the injector 11 into the infusion bag 12. Meanwhile, the second control unit 500 opens the garbage lid 132a, and drops and discards the vial 10B into the garbage storage chamber 13a using the first robot arm 21. In addition, the second control unit 500 allows the second robot arm 22 to move the injector 11 to the injection needle mounting and release device 43, to mount the cap to the injection needle 11 c of the injector 11 and then drop and discard the injector 11 into the garbage storage chamber 13a.

Next, the second control unit 500 reads, from the data storage unit 504, the various images photographed by the injector confirmation camera 42 and the like, and displays those images on the touch panel monitor 14. As a result, the user is able to inspect the suitability of the co-injection process while viewing the touch panel monitor 14.

Note that it is also conceivable that the drug contained in the vial 10B is a drug such as a liquid drug that does not require dissolving. In this case, the co-injection process is the same as the co-injection process for a case in which the drug contained in the vial 10B is a powdered medicine or other such drug that requires dissolving with the exception that the injection step of suctioning the infusion liquid from the infusion bag 12 and injecting it into the vial 10B and the step of stirring the vial 10B are not executed, and therefore explanation thereof is omitted.

Here, in the co-injection process using the vial 10B executed with the co-injection device 1, a suction step for suctioning a liquid drug from inside the vial 10B using the injector 11, and an injection step for injecting the liquid drug into the infusion bag 12 using the injector 11 are executed. Moreover, if the vial 10B contains a powdered medicine, a suction step for suctioning an infusion liquid from the infusion bag 12 using the injector 11, and an injection step for injecting the infusion liquid into the vial 10B using the injector 11 are also executed.

Here, as described above, a rubber stopper 12C is provided at the opening of the vial 10B to thereby seal the vial 10B in an airtight manner. Furthermore, if the injection step of injecting the infusion liquid into the vial 10B using the injector 11 and the suction step of suctioning a liquid drug from the vial 10B are executed, the injection needle 11c of the injector 11 punctures the rubber stopper 12C of the vial 10B. Likewise, a rubber stopper 12A is provided at the opening of the infusion bag 12 to thereby seal the infusion bag 12 in an airtight manner. Furthermore, if the injection step of injecting the infusion liquid into the infusion bag 12 using the injector 11 and the suction step of suctioning the liquid drug from the infusion bag 12 are executed, the injection needle 11 c of the injector 11 punctures the rubber stopper 12A of the infusion bag 12. An explanation is given below using, as examples, a case for which the injector 11 is used to inject the infusion liquid into the vial 10B, and a case for which the injector 11 is used to inject liquid drugs from the vial 10B. However, the case in which the injector 11 is used to inject the infusion liquid into the infusion bag 12, and the case in which the injector 11 is used to inject liquid drugs from the infusion bag 12 are also similar.

Ordinarily, when the injector 11 is used to inject the infusion liquid into the vial 10B, a positive pressure is formed inside the vial 10B, and, for example, the resistance when using the injector 11 to inject the infusion liquid into the vial 10B increases. Moreover, when a positive pressure is formed inside the vial 10B, there is a concern that liquid inside the vial 10B could leak out when the vial 10B is punctured by the injection needle 11 c and the like. Therefore, it is conceivable that when the injector 11 is used to inject the infusion liquid into the vial 10B, the suctioning of air from the vial 10B and the injection of the infusion liquid into the vial 10B are alternately executed.

Moreover, when the injector 11 is used to suction a liquid drug from the vial 10B, a negative pressure is formed in the vial 10B, and for example, when the injector 11 is used to suction the liquid drug from the vial 10B, the resistance becomes larger. Therefore, it is conceivable that when the injector 11 is used to suction liquid drugs from the 10B, the suctioning of liquid drugs from the vial 10B and the injection of air into the vial 10B are alternately executed.

However, the operation of suctioning air when the injector 11 is used to inject infusion liquid and the operation of injecting air when the injector 11 is used to suction liquid drugs are troublesome, and can lead to a delay in the processing time. Moreover, when the injection needle 11c of the injector 11 repeatedly punctures the rubber stopper 10C of the vial 10B, there is a concern of occurrence of coring. Note that the coring is a phenomenon in which a fragment (core) of the rubber stopper 10C is cut out by the injection needle 11 c when the injection needle 11 c punctures the rubber stopper 10C. When the coring occurs, there is a concern that liquid could leak from the vial 10B, and also a concern that a fragment of the rubber stopper 10C could become mixed with the liquid in the vial 10B.

In contrast, in the co-injection device 1, an injection needle 11c configured as described below is used, and as a result, the injection step, the suction step, and the like are simplified, and the process time can be reduced. Moreover, the occurrence of coring is also suppressed because the number of times that the injection needle 11c punctures the rubber stopper 10C of the vial 10B is reduced.

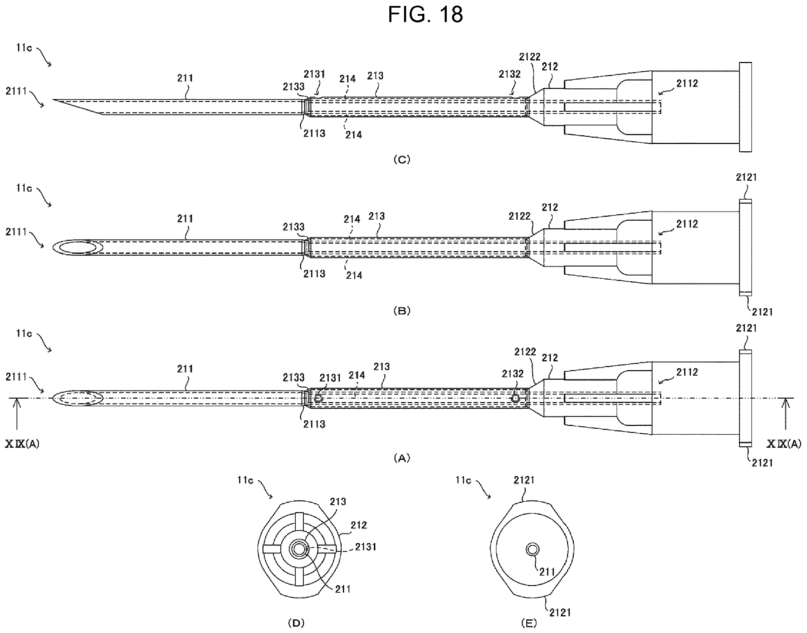

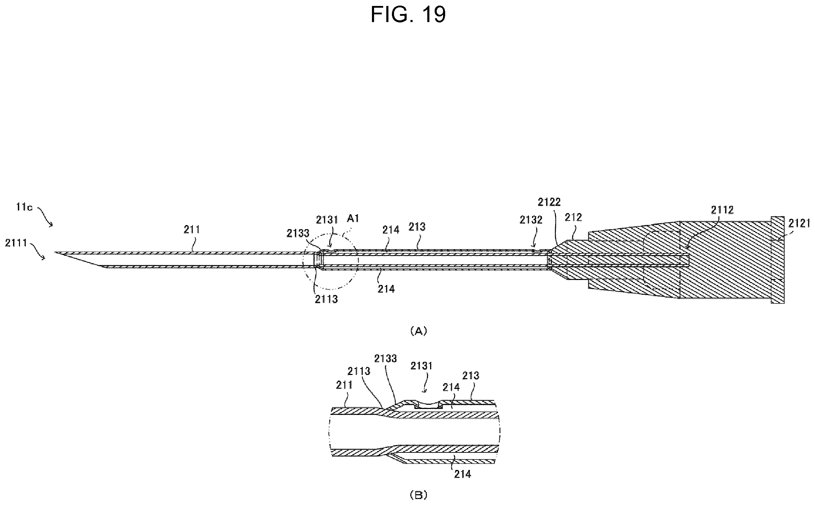

First, an example of the injection needle 11 c of the injector 11 is described with reference to FIG. 18(A) to FIG. 18(E), FIG. 19(A) and FIG. 19(B). Here, FIG. 18(A) is a plan view of the injection needle 11 c, FIG. 18(B) is a bottom view of the injection needle 11 c, FIG. 18(C) is a front elevation view of the injection needle 11c, FIG. 18(D) is a left side view of the injection needle 11c, and FIG. 18(E) is a right side view of the injection needle 11c. Moreover, FIG. 19(A) is a cross-sectional view along the arrow XIX(A)-XIX(A) of FIG. 18(A), and FIG. 19(B) is an enlarged view of the region A1 of FIG. 19(A).

As shown in FIG. 18(A) to FIG. 18(E), FIG. 19(A) and FIG. 19(B), the injection needle 11c is provided with a needle tube 211, a needle base 212, and a ventilation part 213. The injection needle 11 c is manufactured such that after the needle tube 211 is inserted into the ventilation part 213, a rear end 2112 of the needle tube 211 is fixed to the needle base 212. For example, the needle tube 211 and the ventilation part 213 are made of metal, and the needle base 212 is made of a resin. Note that one or both of the needle tube 211 and the ventilation part 213 may also be made of a resin.

A tip end 2111 of the needle tube 211 is a pointed shape capable of puncturing the rubber stopper 10C of the vial 10B. The rear end 2112 of the needle tube 211 is fixed to the needle base 212 in a state of being inserted into the needle base 212. For example, the needle tube 211 is adhered to the needle base 212 by coating the needle tube 211 with an adhesive, and then inserting the rear end 2112 into the needle base 212. Moreover, it is also conceivable that the rear end 2112 of the needle tube 211 is screwed onto the needle base 212. Furthermore, it is also conceivable that the rear end 2112 of the needle tube 211 is fixed by press-fitting it into the needle base 212.

A liquid flow channel for the infusion liquid or liquid drugs or the like suctioned or injected by the injector 11 is formed in the needle tube 211. Moreover, a first taper part 2113 inclined inward from the tip end 2111 side towards the rear end 2112 side is provided at the outer circumferential surface of the needle tube 211. Note that another embodiment that omits the first taper part 2113 is conceivable.

The needle base 212 retains the rear end 2112 side of the needle tube 211, and also sandwiches the ventilation part 213 between the needle base 212 and the needle tube 211. The needle base 212 is detachably mounted to the syringe 11 a, and has a projection 2121 that functions as a male screw that can be screwed into female screw provided on the syringe 11 a. The needle base 212 is mounted to the syringe 11 a by inserting the projection 2121 into the female screw of the syringe 11 a and rotating the projection 2121 in one direction, and the needle base 212 is removed from the syringe 11 a by rotating the projection 2121 in the other direction.

The ventilation part 213 is a tube-shaped member having a first opening 2131, a second opening 2132, and a second taper part 2133. Here, the inner diameter of the ventilation part 213 is larger than the outer diameter of the needle tube 211. In this manner, when the needle tube 211 is inserted into the ventilation part 213, a gap 214 is formed between the outer circumferential surface of the needle tube 211 and the inner circumferential surface of the ventilation part 213. Note that the gap 214 forms a concentric shape with the needle tube 211 and the ventilation part 213. For example, the thickness of the needle tube 211 is 0.1 mm, the thickness of the ventilation part 213 is 0.1 mm, and the thickness of the gap 214 is 0.2 mm.

The first opening 2131 and the second opening 2132 are formed through burring in the circumferential surface of the ventilation part 213, and are through-holes communicating the inside and the outside of the ventilation part 213 (see FIG. 19(B)). Note that the respective quantities of the first opening 2131 and the second opening 2132 are not limited to 1, and 2 or more may be provided at prescribed positions in the longitudinal direction and/or the circumferential direction of the ventilation part 213.

The first opening 2131 is formed at a predetermined first position located at the rear end 2112 side apart from the tip end 2111 of the needle tube 211. In addition, the second opening 2132 is formed at a predetermined second position located at the rear end 2112 side of the needle tube 211 and separated from the tip end 2111 more than the first position at which the first opening 2131 is formed. Note that the first opening 2131 and the second opening 2132 are positioned closer to the tip end 2111 of the needle tube 211 than the needle base 212. With the ventilation part 213 configured in this manner, a ventilation path is formed through a simple configuration of the first opening 2131, the gap 214, and the second opening 2132, and air distribution is possible between the first position and the second position. Namely, the ventilation part 213 communicates the first position at which the first opening 2131 is formed and the second position at which the second opening 2132 is formed with the gap 214 at the outside of the needle tube 211.

The second taper part 2133 is inclined inward from the rear end 2112 side towards the tip end 2111 side. In addition, the ventilation part 213 is mounted to the needle tube 211 with the second taper part 2133 in a state of facing the tip end 2111 of the needle tube 211. Here, the inner diameter of the end part of the second taper part 2133 of the ventilation part 213 is smaller than the maximum outer diameter of the first taper part 2113 of the needle tube 211. Therefore, when the ventilation part 213 is mounted to the needle tube 211, the end part of the second taper part 2133 abuts the first taper part 2113, and thereby positioning of the ventilation part 213 and the needle tube 211 is performed.

Here, the abutting location between the first taper part 2113 and the second taper part 2133 that becomes the boundary portion between the needle tube 211 and the ventilation part 213 is accommodated inside the needle tube 211 when viewed from the tip end 2111 side of the needle tube 211. Therefore, coring, which may occur when the boundary portion between the needle tube 211 and the ventilation part 213 contacts the rubber stopper 10C when the injection needle 11c punctures the rubber stopper 10C of the vial 10B, is prevented.

Moreover, a taper part 2122 inclined inward from the rear end 2112 side towards the tip end 2111 side is provided at the needle base 212. When the needle tube 211 is mounted to the needle base 212, the taper part 2122 abuts the rear end of the ventilation part 213. Namely, the ventilation part 213 is held between the first taper part 2113 of the needle tube 211 and the taper part 2122 of the needle base 212. Accordingly, there is no need to fix the ventilation part 213 to the needle tube 211 or the needle base 212 through adhesive, soldering, welding, or the like.

### Injection Step

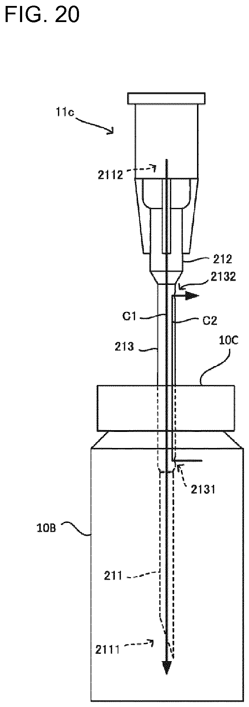

Next, the injection step, which is performed in the co-injection process executed by the co-injection device 1 using the injector 11 to which the injection needle 11c is mounted, is described with reference to FIG. 20. The injection step is executed by the second control unit 500 controlling the first robot arm 21, the second robot arm 22, and the like. Here, when the relevant process is executed, the second control unit 500 is one example of an injection control unit.

As shown in FIG. 20, in the injection step, first a puncture step is performed in which the rubber stopper 10C of the vial 10B is punctured with the injection needle 11 c of the injector 11 using the first robot arm 21 and the second robot arm 22. In the puncture step, the injection needle 11c of the injector 11 punctures the rubber stopper 10C until the first position at which the first opening 2131 of the ventilation part 213 is formed is positioned inside the vial 10B, and the second position at which the second opening 2132 of the ventilation part 213 is formed is positioned outside of the vial 10B. In other words, the injection needle 11 c of the injector 11 punctures the rubber stopper 10C of the vial 10B as far as a position between the first position and the second position. There, the puncture step is an example of a first puncture step, and in this manner, the inside and outside of the vial 10B are communicated at the ventilation part 213 by the first opening 2131, the gap 214, and the second opening 2132.

Furthermore, with the injection needle 11 c of the injector 11 being made to puncture the rubber stopper 10C of the vial 10B by the puncture step, the injection step for pushing in the plunger 11 b of the injector 11 using the second robot arm 22 is executed. With the injection step, the infusion liquid contained in the syringe 11 a of the injector 11 flows in the direction of the arrow C1 inside the needle tube 211 of the injection needle 11c of the injector 11 and is injected into the vial 10B. At this time, the air inside the vial 10B is pushed out by the injection of the infusion liquid into the vial 10B, flows in the direction of the arrow C2 inside the ventilation part 213, and is discharged outside the vial 10B.

As a result, even if the infusion liquid is injected into the vial 10B, a positive pressure is not formed inside the vial 10B, and the plunger 11 b of the injector 11 can be pushed as it is. Through this, there is no need to alternately repeat the discharging of air from the inside of the vial 10B to the outside and the injection of the infusion liquid into the vial 10B using the injector 11. Accordingly, the injection step using the injector 11 is simplified, and therefore the processing time required for the injection step is reduced. Moreover, because there is no need to repeatedly puncture the rubber stopper 10C of the vial 10B with the injection needle 11 c, the occurrence of coring is suppressed. Note that a case of operating the injector 11 through the co-injection device 1 was given as an example here, but a case of a human operating the injector 11 is also similar.

### Suction Step

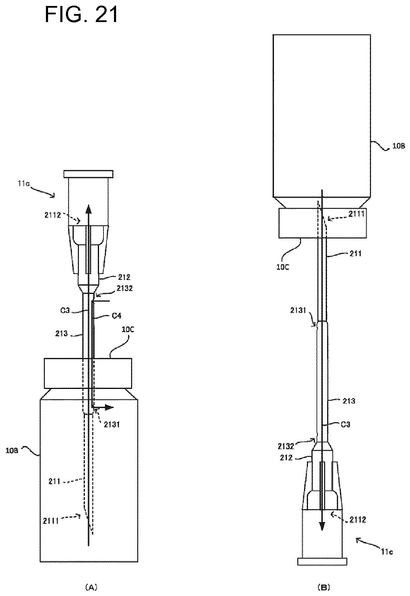

Next, the suction step performed using the injector 11 with the injection needle 11 c in the co-injection process executed by the co-injection device 1 is described with reference to FIG. 21 (A). The suction step is executed by controlling the first robot arm 21, the second robot arm 22, and the like by means of the second control unit 500. Here, when the relevant process is executed, the second control unit 500 is one example of a suction control unit.

As shown in FIG. 21 (A), in the suction step, first a puncture step is executed by the first robot arm 21 and the second robot arm 22 to puncture the rubber stopper 10C of the vial 10B with the injection needle 11c of the injector 11. In the puncture step, the injection needle 11c of the injector 11 punctures the rubber stopper 10C until the first position at which the first opening 2131 of the ventilation part 213 is formed is positioned inside the vial 10B, and the second position at which the second opening 2132 of the ventilation part 213 is formed is positioned outside of the vial 10B. In other words, the injection needle 11 c of the injector 11 punctures the rubber stopper 10C of the vial 10B as far as a position between the first position and the second position. Here, the puncture step is an example of a third puncture step, and in this manner, the inside and outside of the vial 10B are communicated at the ventilation part 213 by the first opening 2131, the gap 214, and the second opening 2132.

Furthermore, with the injection needle 11 c of the injector 11 being made to puncture the rubber stopper 10C of the vial 10B to a position between the first position and the second position, operation for pulling out the plunger 11 b of the injector 11 is executed using the second robot arm 22. Through this, the liquid drug contained in the vial 10B flows in the direction of the arrow C3 inside the needle tube 211 of the injection needle 11c of the injector 11, and is suctioned to the syringe 11 a of the injector 11. At this time, the liquid drug is suctioned from the vial 10B. However, air outside of the vial 10B flows in the direction of the arrow C4 inside the ventilation part 213 and is injected into the vial 10B.

As a result, even when a liquid drug is suctioned from the vial 10B, a negative pressure is not formed inside the vial 10B, and the plunger 11 b of the injector 11 can be pulled out as it is. Through this, there is no need to alternately repeat the injection of air into the vial 10B and the suction of the liquid drug from the vial 10B using the injector 11. Accordingly, the suction step of the injector 11 is simplified, and as a result, the process time required for the suction step is reduced. Moreover, because there is no need to repeatedly puncture the rubber stopper 10C of the vial 10B with the injection needle 11c, the occurrence of coring can be suppressed. Note that here, a case of operating the injector 11 using the co-injection device 1 was described, but a case of a human operating the injector 11 is also similar.

Moreover, in the example shown in FIG. 21 (A), the orientation of the injector 11 and the vial 10B in the suction step is such that the tip end 2111 of the injection needle 11 c of the injector 11 faces the vertically downward direction, and the mouth part of the vial 10B faces the vertically upward direction. However, it is also conceivable that as shown in FIG. 21 (B), the orientation of the injector 11 and the vial 10B in the suction step is such that the mouth part of the vial 10B faces the vertically downward direction, and the tip end 2111 of the injection needle 11 c of the injector 11 faces the vertically upward direction. In this case, in the puncture step, the injection needle 11 c of the injector 11 punctures the rubber stopper 10C of the vial 10B as far as a position further to the tip end 2111 side than a first opening 3111 of the ventilation part 213. Here, the puncture step is one example of a second puncture step, and through this step, even if the plunger 11 b of the injector 11 is pulled out, liquid drugs do not leak out from the ventilation part 213. Note that in this case, it is conceivable that suctioning of the liquid drugs from the vial 10B and the injection of air into the vial 10B could be alternately performed. Moreover, if the amount of liquid drugs suctioned from the vial 10B is minimal, or the like, the suctioning of liquid drugs from the vial 10B and the injection of air into the vial 10B may not be alternately performed if the inside of the vial 10B does not become a negative pressure at or above a predetermined tolerance value.

In the present embodiment, the first position and the second position of the injector needle 11 are positioned further at the tip end 2111 side of the needle base 212 than the needle tube 211. In contrast, it is also conceivable that the first position and the second position of the injector needle 11 could be positioned at the needle base 212 side retaining the needle tube 211. However, in this case, when the injection needle 11c punctures the rubber stopper 10C of the vial 10B, it must puncture as far as the needle base 212 where the outer diameter is larger than the needle tube 211. As a result, coring occurs more easily in comparison to the case in which only the needle tube 211 punctures the rubber stopper 10C. For example, when a configuration in which the needle base 212 is inserted into the ventilation part 213 is considered, the outer diameter of the ventilation part 213 must be made larger in comparison to the configuration of the present embodiment in which the needle tube 211 is inserted into the ventilation part 213, and thus coring more easily occurs. In contrast, in the injector 11, the first position and the second position are positioned at the tip end 2111 side of the needle tube 211 closer to the needle base 212, and therefore when the injection needle 11c punctures the rubber stopper 10C of the vial 10B, there is no need to puncture as far as the needle base 212, and the occurrence of coring is more effectively suppressed.

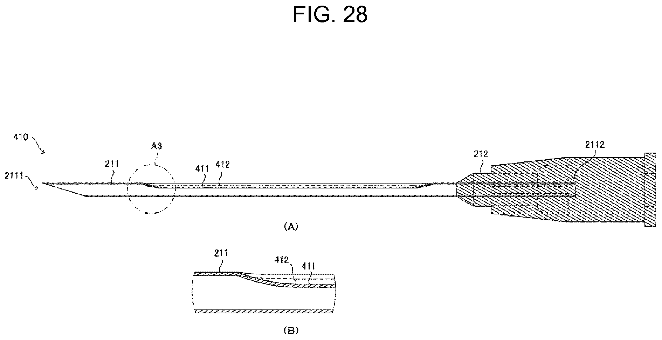

Moreover, as described later, it is also conceivable that in place of the ventilation part 213, a groove part 411 (see FIG. 28(A) and the like) could be formed in the outer circumferential surface of the needle tube 211. In contrast, a configuration for which the groove part 411 is formed in the needle base 212 is also conceivable, but in this case, when the injection needle 11 c punctures the rubber stopper 10C of the vial 10B, the injection needle 11 c must puncture as far as the needle base 212 having the outer diameter larger than that of the needle tube 211, and thus coring easily occurs. However, if the first position and the second position are positioned closer to the tip end 2111 of the needle tube 211 than the needle base 212, the groove part 411 is formed closer to the tip end 2111 than the needle base 212, and therefore when the injection needle 11c punctures the rubber stopper 10C of the vial 10B, the injection needle 11c does not have to puncture as far as the needle base 212, and the occurrence of coring is effectively suppressed.

Incidentally, the injection needles 11c shown in FIG. 18(A) to FIG. 18(E) are merely examples, and other examples of the injection needle 11c are described below in the Second Embodiment to the Eighth Embodiment.

### Second Embodiment

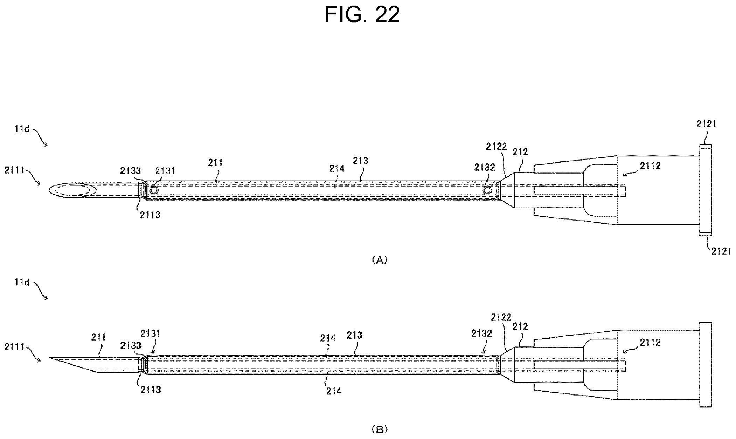

FIG. 22(A) and FIG. 22(B) are images showing an injection needle 11 d, which is a modified example of the injection needle 11 c according to the first embodiment. Note that the same reference numerals as those used for the injection needle 11 c are given to components of the injection needle 11 d having the same configuration as the components of the injection needle 11c, and explanations thereof are omitted. Here, FIG. 22(A) is a plan view of the injection needle 11 d, and FIG. 22(B) is a front elevation view of the injection needle 11 d. Note that the right side view and the left side view of the injection needle 11 d are the same as those for the injection needle 11c, and therefore descriptions thereof are omitted.

The injection needle 11d shown in FIG. 22(A) and FIG. 22(B) has a shorter interval distance between the tip end 2111 of the needle tube 211 and the first opening 2131 compared to the injection needle 11c shown in FIG. 18(A) and FIG. 18(B). As a result, if a liquid is contained in the vial 10B as far as a position close to the rubber stopper 10C, the injection step and the suction step can be performed using the injector 11 with the injection needle 11 d even if the injection needle 11 d of the injector 11 is facing the vertically downward direction, and the rubber stopper 10C of the vial 10B is facing the vertically upward direction. In this manner, the interval distance between the tip end 2111 of the needle tube 211 and the first opening 2131 of the injection needle 11c and the injection needle 11d may be optionally determined in advance in accordance with the objective.

### Third Embodiment

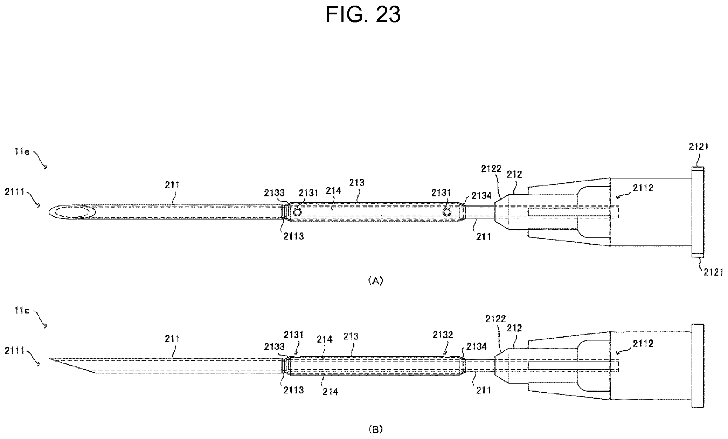

FIG. 23(A) and FIG. 23(B) are images showing an injection needle 11 e as another aspect of the injection needle 11c.Note that the same reference numerals as those used for the injection needle 11 c are given to components of the injection needle 11e having the same configuration as the components of the injection needle 11c, and explanations thereof are omitted. Here, FIG. 23(A) is a plan view of the injection needle 11e, and FIG. 23(B) is a front elevation view of the injection needle 11e. Note that the right side view and the left side view of the injection needle 11e are the same as those for the injection needle 11c, and therefore descriptions thereof are omitted.

In the injection needle 11 e shown in FIG. 23(A) and FIG. 23(B), a taper part 2134 inclined to the inside from the rear end 2112 side to the tip end 2111 side is provided on the outer circumferential surface of the ventilation part 213. Furthermore, in the ventilation part 213, the end part of the taper part 2134 is fixed to the outer circumferential surface of the needle tube 211 through adhesive, soldering, welding, or the like.

Moreover, the needle tube 211 is fixed to the needle base 212 between, for example, the ventilation part 213 and the needle base 212 by adhesive, soldering, welding, or the like. Note that similar to the first embodiment, it is also conceivable that the rear end 2112 of the needle tube 211 is inserted and affixed into the needle base 212 with the rear end 2112 being coated with an adhesive, that the needle tube 211 is screwed onto the needle base 212, or that the rear end 2112 of the needle tube 211 is press fit into the needle base 212 and secured.

### Fourth Embodiment

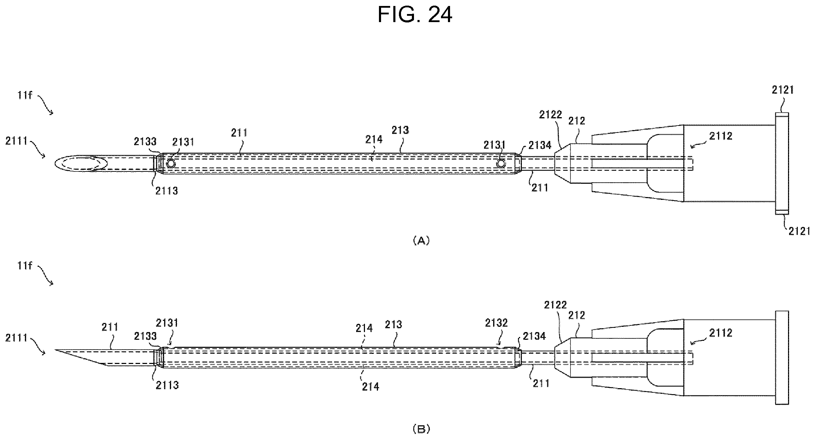

FIG. 24(A) and FIG. 24(B) are images showing an injection needle 11f as a modified example of the injection needle 11 e according to the third embodiment. Note that the same reference numerals as those used for the injection needle 11 e are given to components of the injection needle 11f having the same configuration as the components of the injection needle 11e, and explanations thereof are omitted. Here, FIG. 24(A) is a plan view of the injection needle 11 f, and FIG. 24(B) is a front elevation view of the injection needle 11 f. Note that the right side view and the left side view of the injection needle 11 f are the same as those for the injection needle 11c, and therefore descriptions thereof are omitted.

The injection needle 11 f shown in FIG. 24(A) and FIG. 24(B) has a shorter interval distance between the tip end 2111 of the needle tube 211 and the first opening 2131 compared to the injection needle 11e shown in FIG. 23(A) and FIG. 23(B). Through this, when a liquid is contained in the vial 10B as far as a position near the rubber stopper 10C, even if the injection needle 11 f of the injector 11 is facing the vertically downward direction, and the rubber stopper 10C of the vial 10B is facing the vertically upward direction, the injection step and the suction step can be performed using the injector 11 with the injection needle 11f mounted thereon. As a result, the interval distance between the tip end 2111 of the needle tube 211 and the first opening 2131 of the injection needle 11e and the injection needle 11 f may be optionally determined in advance according to the objective.

### Fifth Embodiment

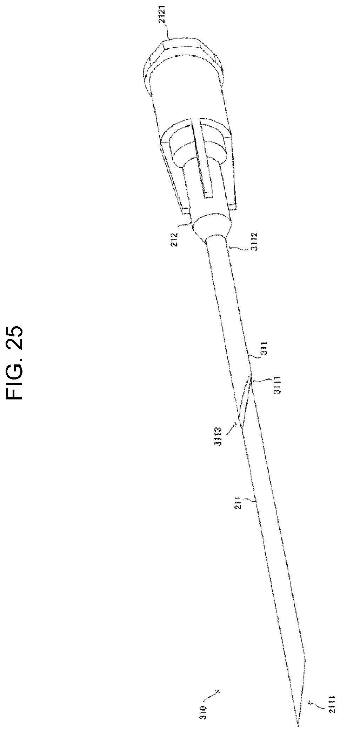

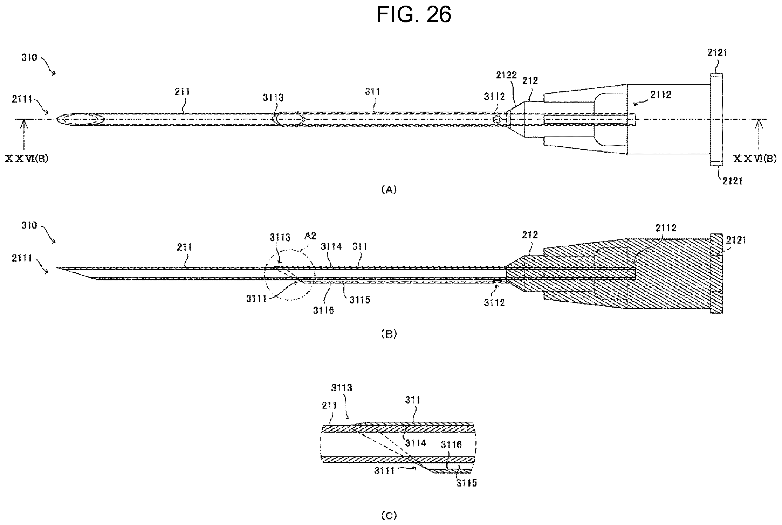

FIG. 25 and FIG. 26(A) to FIG. 26(C) are images showing an injection needle 310, which is another example of the injection needle 11c of the injector 11. Note that the same reference numerals as those used for the injection needle 11 c are given to components of the injection needle 310 having the same configuration as the components of the injection needle 11c, and explanations thereof are omitted. Here, FIG. 25 is a perspective view of the injection needle 310. Moreover, FIG. 26(A) is a plan view of the injection needle 310, FIG. 26(B) is a cross-sectional view along the arrow XXVI(B)-XXVI(B) of FIG. 26(A), and FIG. 26(C) is an enlarged view of the area A2 of FIG. 26(B).

As shown in FIG. 25 and FIG. 26(A) to FIG. 26(C), the injection needle 310 has a ventilation part 311 in place of the ventilation part 213 of the injection needle 11c.The ventilation part 311 is a tube-shaped member provided with a first opening 3111 at a position corresponding to the first position, and a second opening 3112 at a position corresponding to the second position.

The first opening 3111 is an open end part at the tip end 2111 side of the ventilation part 311. The first opening 3111, which is an open end part, is an opening formed by not providing an end face of the tip end 2111 side in the axial direction of the ventilation part 311. Moreover, similar to the second opening 2132 of the ventilation part 213, the second opening 3112 is a through-hole communicating the inside and outside of the ventilation part 311, and is formed in the circumferential surface of the ventilation part 311. Note that similar to the first opening 2131 of the ventilation part 213, the first opening 3111 is a through-hole communicating the inside and outside of the ventilation part 311, and as another embodiment, it is also conceivable that the second opening 3112 is an open end part at the rear end 2112 side of the ventilation part 311.

Here, the ventilation part 311 has an inner diameter larger than the outer diameter of the needle tube 211. Furthermore, with the ventilation part 311, a first region 3114, which is a portion of the inner circumferential surface, is fixed to the needle tube 211 in a state of being circumscribed to the outer circumferential surface of the needle tube 211. Through this, inside the ventilation part 311, a gap 3115 is interposed between the inner circumferential surface of the ventilation part 311 and the outer circumferential surface of the needle tube 211 at a second region 3116 excluding the first region 3114. In the ventilation part 311, the open end part at the tip end 2111 side of the gap 3115 is the first opening 3111. Note that the ventilation part 311 is fixed by an adhesive, soldering, welding, or the like.

Furthermore, in the ventilation part 311 configured in this manner, a ventilation path communicating the first position and the second position is formed outside the needle tube 211 by the first opening 3111, the gap 3115, and the second opening 3112. As a result, similar to the first embodiment, the discharge of air from the vial 10B and the injection of air into the vial 10B are performed through the ventilation part 311 in the injection step, the suction step, and the like, which are performed using the injector 11 with the injection needle 310 mounted thereon.

Here, in the injection needle 310, a third taper part 3113, for which the wall thickness increases towards the outside in a direction from the tip end 2111 side to the rear end 2112 side, is provided at one end of the ventilation part 311. In the outer circumferential direction of the ventilation part 311, the third taper part 3113 includes a first region 3114 circumscribing to the outer circumferential surface of the needle tube 211, and a second region 3116 having a gap 3115 interposed between the outer circumferential surface of the needle tube 211 at a position opposing the first region 3114 and the ventilation part 311. In other words, a gap is not formed between the inner circumferential surface of the first region 3114 and the outer circumferential surface of the needle tube 211.

Moreover, in the third taper part 3113, an end part at the tip end 2111 side of the first region 3114 is positioned closer to the tip end 2111 than the end part of the tip end 2111 side of the second region 3116. As a result, in the ventilation part 311 of the injection needle 310, the first region 3114 punctures the rubber stopper 10C of the vial 10B ahead of the second region 3116 where the gap 3115 is formed. Accordingly, the injection needle 310 is more easily inserted into the rubber stopper 10C in comparison to a case in which the first region 3114 and the second region 3116 having the gap 3115 simultaneously puncture the rubber stopper 10C.

Furthermore, in the third taper part 3113, as shown in FIG. 26(C), the inclination angle of the outer circumferential surface of the ventilation part 311 with respect to the outer circumferential surface of the needle tube 211 in the first region 3114 is smaller than the inclination angle of the outer circumferential surface of the ventilation part 311 with respect to the outer circumferential surface of the needle tube 211 in the second region 3116. More specifically, the inclination angle of the third taper part 3113 with respect to the outer circumferential surface of the needle tube 211 becomes smaller in a direction from the first region 3114 to the second region 3116 in the circumferential direction of the ventilation part 311. As a result, when the first region 3114, which is the tip end of the ventilation part 311 and at which the gap 3115 is not formed, punctures the rubber stopper 10C, the pointedness increases, and the insertion resistance decreases, and along with this, when the second region 3115 at which the gap 3115 is formed punctures the rubber stopper 10C, the pointedness thereof becomes less than that of the first region 3114, and the occurrence of coring at the second region 3115 is suppressed.

### Sixth Embodiment

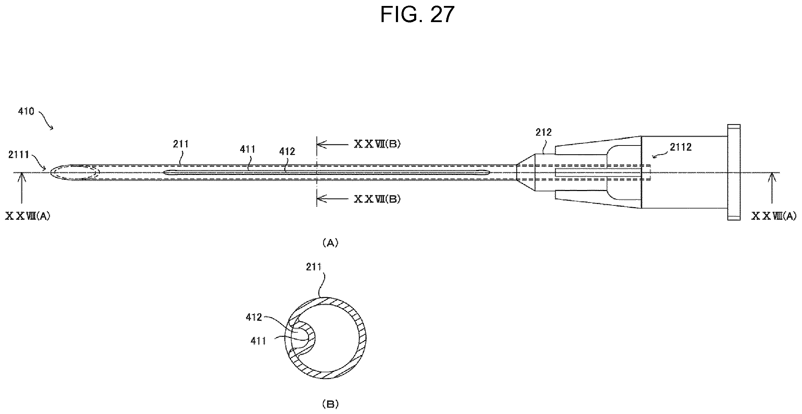

FIG. 27(A), FIG. 27(B), FIG. 28(A), and FIG. 28(B) are images showing an injection needle 410 as another example of the injection needle 11 c of the injector 11. Note that the same reference numerals as those used for the injection needle 11 c are given to components of the injection needle 410 having the same configuration as the components of the injection needle 11c, and explanations thereof are omitted. Here, FIG. 27(A) is a plan view of the injection needle 410, and FIG. 27(B) is a cross-sectional view along the arrow XXVII(B)-XXVII(B) of FIG. 27(A). Moreover, FIG. 28(A) is a cross-sectional view along the arrow XXVIII(A)-XXVIII(A) of FIG. 27(A), and FIG. 28(B) is an enlarged view of section A3 of FIG. 28(A).

As shown in FIG. 27(A), FIG. 27(B), FIG. 28(A), and FIG. 28(B), the injection needle 410 has a groove part 411 provided on the outer circumferential surface of the needle tube 211. The groove part 411 is formed from the first position to the second position on the outer circumferential surface of the needle tube 211. Note that as described above, the first position is a position on the needle tube 211 positioned apart from the tip end 2111 toward the rear end 2112 side, and the second position is a position separated from the first position toward the rear end 2112 side of the needle tube 211.

In the injection needle 410 configured in this manner, an internal space 412 of the groove part 411 is formed as a ventilation path communicating the first position and the second position at the outside of the needle tube 211. As a result, similar to the first embodiment, in the injection step, the suction step, and the like to be performed using the injector 11 with the injection needle 410 mounted thereon, the discharging of air from the vial 10B and the injection of air into the vial 10B are performed through the groove part 411.

Furthermore, in the injection needle 410, because the groove part 411 need only be formed in the needle tube 211, the embodiment of the present invention can be realized with a minimal number of components. Moreover, the groove part 410 is housed inside the needle tube 211 when viewed from the tip end 2111 side of the needle tube 211, and therefore the occurrence of coring attributed to the groove part 410 is suppressed. Note that the length of the groove part 411 of the injection needle 410 may be optionally determined in accordance with the objective.

### Seventh Embodiment

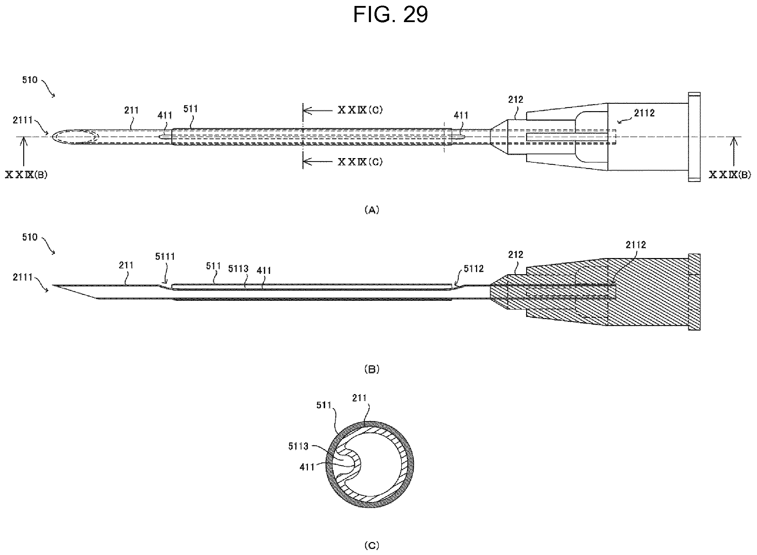

FIG. 29(A) to FIG. 29(C) are images showing an injection needle 510 as a modified example of the injection needle 410 according to the sixth embodiment. Note that the same reference numerals as those used for the injection needle 410 are given to components of the injection needle 510 having the same configuration as the components of the injection needle 410, and explanations thereof are omitted. Here, FIG. 29(A) is a plan view of the injection needle 510, FIG. 29(B) is a cross-sectional view along the arrow XXIX(B)-XXIX(B) of FIG. 29(A), and FIG. 29(C) is a cross-sectional view along the arrow XXIX(C)-XXIX(C) of FIG. 29(A).

As shown in FIG. 29(A) to FIG. 29(C), the injection needle 510 has the groove part 411 provided in the outer circumferential surface of the needle tube 211, and a tube-shaped member 511 which is provided across a portion of the range of the groove part 411 and allows a gap to be formed between the inner circumferential surface of the tube-shaped member 511 and the groove part 411 when the needle tube 211 is inserted internally. Here, the groove part 411 and the tube-shaped member 511 are an example of the ventilation part.

Furthermore, in the tube-shaped member 511, a first opening 5111 is provided at a position corresponding to the first position, and a second opening 5112 is provided at a position corresponding to the second position. The first opening 5111 and the second opening 5112 are an open end part at the tip end 2111 side and an open end part at the rear end 2112 side of a gap 5113 interposed between the inner circumferential surface of the tube-shaped member 511 and the outer circumferential surface of the needle tube 211. Note that the first opening 5111 and the second opening 5112, which are open parts, are openings formed by not providing an end face at the tube-shaped member 511 in the axial direction of the tube-shaped member 511.

In the injection needle 510 configured in this manner, the gap 5113 formed between the inner circumferential surface of the tube-shaped member 511 and the groove part 411 on the outer circumferential surface of the needle tube 211 is formed as a ventilation path communicating the first position and the second position. In this manner, similar to the first embodiment, in the injection step, the suction step, and the like to be performed using the injector 11 with the injection needle 510 mounted thereon, the discharging of air from the vial 10B and the injection of air into the vial 10B are performed through the groove part 411.

Furthermore, in the injection needle 510, the gap 5113 between the tube-shaped member 511 and the groove part 411 indenting to the inside of the needle tube 211 is used as a ventilation path, and therefore, even if the inner diameter and the outer diameter of the tube-shaped member 511 are small, the gap 5113 can be secured as a ventilation path. Accordingly, in the injection needle 510, there is no need to secure a gap that serves as a ventilation path between the outer circumferential surface of the needle tube 211 and the inner circumferential surface of the tube-shaped member 511, and therefore the outer diameter of the tube-shaped member 511 can be made small, and the occurrence of coring can be suppressed. For example, the outer circumferential surface of the needle tube 211 and the inner circumferential surface of the tube-shaped member 511 may also be tightly adhered.

Note that another embodiment is also conceivable in which the tube-shaped member 511 is provided extending the entire range of the groove part 411, and a gap is formed between the groove part 411 and the inner circumferential surface of the tube-shaped member 511 when the needle tube 211 is inserted internally. In this case, another embodiment is conceivable in which the tube-shaped member 511 has a first opening and a second opening which are the first position and the second position, respectively and are formed at positions on the circumferential surface of the tube-shaped member 511 opposing the groove part 411, and communicate the inside and outside of the tube-shaped member 511. In this case as well, a ventilation path is formed by the first opening, the groove part 411, and the second opening, and therefore the outer diameter of the tube-shaped member 511 can be made smaller in comparison to the ventilation part 213 of the first embodiment, and the occurrence of coring can be suppressed.

### Eighth Embodiment

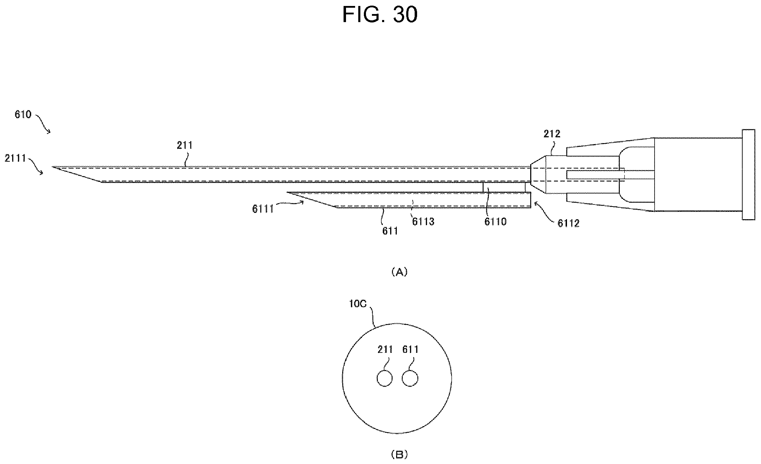

FIG. 30(A) is an image showing an injection needle 610 as another example of the injection needle 11 c of the injector 11, and FIG. 30(B) is a schematic view showing a state in which the injection needle 610 is puncturing the rubber stopper 10C. Note that the same reference numerals as those used for the injection needle 11 c are given to components of the injection needle 610 having the same configuration as the components of the injection needle 11c, and explanations thereof are omitted.

As shown in FIG. 30(A), the injection needle 610 has the needle tube 211, and a ventilation needle tube 611 fixed to the needle tube 211 via a connection 6110 in a state of being parallel to the needle tube 211. Here, the ventilation needle tube 611 is an example of the ventilation part. Note that the length and position of the ventilation needle tube 611 with respect to the injection needle 610 may be optionally determined in advanced in accordance with the objective.

The connection 6110 is a fixing part for the ventilation needle tube 611 interposed when the ventilation needle tube 611 is connected to the needle tube 211 by an adhesive, soldering, welding, or the like at a third position located closer to the rear end 2112 than the first position. For example, the connection 6110 is an adhesive, solder, welding material, spacer member, or the like. Accordingly, the needle tube 211 and the ventilation needle tube 611 are separated in direction vertical to the longitudinal direction of the injection needle 610. Note that in the injection needle 610, the connection 6110 is provided between the first position and the second position.

Moreover, in the ventilation needle tube 611, a first opening 6111 is provided at a position corresponding to the first position, and a second opening 6112 is provided at a position corresponding to the second position. The first opening 6111 is an open end part at the tip end 2111 side of the ventilation needle tube 611, and the second opening 6112 is an open end part at the rear end 2112 side of the ventilation needle tube 611. Furthermore, a ventilation path 6113 for communicating the first opening 6111 and the second opening 6112 is formed in the ventilation needle tube 611.

In the injection step, the suction step, and the like to be performed using the injector 11 mounted with the injection needle 610 configured as described above, discharging of air from the vial 10B and injection of air into the vial 10B are performed through the ventilation path 6113 of the ventilation needle tube 611. Here, in the injection needle 610, the needle tube 211 and the ventilation needle tube 611 are connected via the connection 6110, and the needle tube 211 and the ventilation needle tube 611 are separating from each other in a direction vertical to the longitudinal direction of the injection needle 610. Therefore, when the needle tube 211 and the ventilation needle tube 611 are puncturing the rubber stopper 10C of the vial 10B, the port for insertion into the rubber stopper 10C of the needle tube 211 and the ventilation needle tube 611 is not connected. Accordingly, by using the injection needle 610, damage to the rubber stopper 10C and the occurrence of coring are suppressed in comparison to a configuration in which the ventilation needle tube 611 is directly fixed to the outer circumferential surface of the needle tube 211.

### Ninth Embodiment

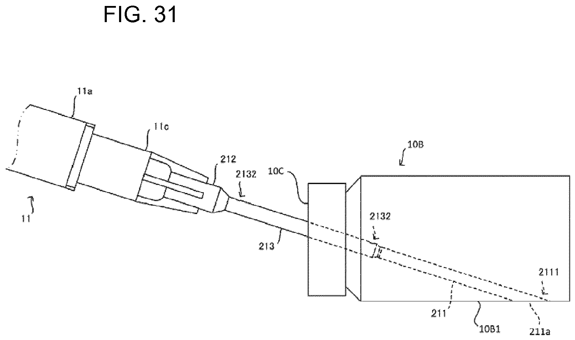

FIG. 31 is an image for explaining another example of the suction step performed using the injection needle 11 c according to the first embodiment of the co-injection device 1.

First, before the suction step is executed, the second control unit 500 allows the injector 11 to move within the photographing range R1 of the injector confirmation camera 42, to photograph the state of the injection needle 11c of the injector 11. At this time, the second control unit 500 is configured to photograph the injection needle 11 c of the injector 11 in a plurality of cases where the positional relationship between the injector confirmation camera 42 and the injection needle 11 c is relatively varied such that the position of the injection needle 11 c to be photographed by the injector confirmation camera 42 differs in the circumferential direction thereof.

Furthermore, the second control unit 500 is configured to detect the orientation of the injection needle 11c with respect to the injector 11 based on the image photographed by the injector confirmation camera 42. More specifically, it is conceivable that the second control unit 500 executes image matching processing to detect the positions of the first opening 2111, the second opening 2112, and the like with respect to the injection needle 11 c. In other words, in the co-injection device 1, the second control unit 500 can detect the orientation of the injection needle 11c using the shape of the ventilation part 213, which is used for forming a ventilation path. Through this, for example, the time and labor required to make a mark on the injection needle 11 c is eliminated. Note that it is also conceivable that the second control unit 500 is configured to orient the injection needle 11c based on the shape of the tip end 2111 of the injection needle 11c.

When the orientation of the injection needle 11 c is specified, in the suction step, the second control unit 500 is configured to initiate suction of the liquid drug from the vial 10B using the injector 11. Of course, because the opening end face of the tip end of the needle tube 211 is inclined, as shown in FIG. 21 (A), when the remaining amount of liquid drug in the vial 10B reaches a preset prescribed amount or less in a state of the tip end 2111 of the injection needle 11 c facing the vertically downward direction and the rubber stopper 10C of the vial 10B facing the vertically upward direction, it becomes difficult to suction all of the liquid drug in the vial 10B. Moreover, as shown in FIG. 21 (B), similarly, even if the tip end 2111 of the injection needle 11c is facing the vertically upward direction, and the rubber stopper 10C of the vial 10B is facing the vertically downward direction, when the remaining amount of liquid drug in the vial 10B reaches a preset prescribed amount or less, it become difficult to suction all of the liquid drug in the vial 10B.

Therefore, when the remaining amount of liquid drug in the vial 10B reaches a prescribed amount or less, the second control unit 500 s configured to control the first robot arm 21, the second robot arm 22, and the like such that the injection needle 11 c of the injector 11 and the vial 10B are oriented as shown in FIG. 31. At this time, the second control unit 500 detects the orientation of the injection needle 11c based on the image photographed by the injector confirmation camera 42 before the suction step is executed, and therefore the orientation of the injector 11c can be adjusted to an optional direction based on the control coordinates of the second robot arm 22 during the detection thereof. More specifically, as shown in FIG. 31, the second control unit 500 is configured to tilt the vial 10B such that a side surface 10B1 of the vial 10B becomes parallel with the horizontal direction, and also tilt the injector 11 such that an opening end face 211 a of the needle tube 211 of the injection needle 11 c abuts the side surface 10B1 of the vial 10B. In this manner, all of the liquid drug inside the vial 10B can be suctioned from the opening end face 211 a of the needle tube 211.

Note that here, a case in which the injection needle 11c is used was described, but the same applies for cases using the other injection needles described in the Second Embodiment to the Eighth Embodiment. For example, when the injection needle 310 according to the Fifth Embodiment is used, it is conceivable that the orientation of the injection needle 310 is detected based on the shape of the third taper part 3113 in the image photographed by the injector confirmation camera 42. Moreover, when the injection needle 410 according to the Sixth Embodiment is used, it is conceivable that the orientation of the injection needle 310 is detected based on the shape of the groove part 411 in the image photographed by the injector confirmation camera 42. Furthermore, when the injector 610 of the Eighth Embodiment is used, it is conceivable that the orientation of the injection needle 310 is detected based on the position of the ventilation needle tube 611 in the circumferential direction of the needle tube 211.

### REFERENCE NUMERALS

1: co-injection device, 10: medicinal container, 11: injector, 11a: syringe main body, 11b: plunger, 11c: injection needle, 11 d: syringe filter, 12: infusion bag, 13: garbage storage chamber door, 15a: IC reader, 21: first robot arm, 22: second robot arm, 25: holding part, 26: holding part, 31: ampoule cutter, 32: stirring device, 33: placement shelf, 33A: rotation placement section, 34: drug reading unit, 35: weighing meter, 36: needle bend detection unit, 37: co-injection communication port, 38: transparent window for needle insertion confirmation, 39: weighing meter, 41: tray confirmation camera, 42: injector confirmation camera, 43: injection needle mounting and release device, 44: needle insertion confirmation camera, 45: sterilization lamp, 100: co-injection control device, 101: tray, 101 a: electronic paper, 101 b : IC tag, 101 c: IC reader, 104: co-injection process chamber, 110: tray conveyance unit, 121: infusion liquid camera, 200: drug loading unit, 300: co-injection processing unit, 400: first control unit, 401: CPU, 402: ROM, 403: RAM, 404: data storage unit, 500: second control unit, 501: CPU, 502: ROM, 503: RAM, 504: data storage unit, 600: higher order system

## Claims

1. An injection needle comprising:
a needle tube; and
a ventilation part communicating, outside the needle tube, a first position positioned nearer to a rear end side than a tip end of the needle tube and a second position positioned nearer to the rear end side than the first position.

2. The injection needle according to claim 1, wherein the ventilation part is a tube-shaped member, and when the needle tube is inserted into the ventilation part, a gap communicating the first position and the second position is formed between an inner circumferential surface of the ventilation part and an outer circumferential surface of the needle tube.

3. The injection needle according to claim 2, wherein an opening communicating the inside and outside of the ventilation part is formed in the ventilation part at one or both of the first position and the second position.

4. The injection needle according to claim 2 or claim 3, wherein an open end part of the gap is positioned at one or both of the first position and the second position of the ventilation part.

5. The injection needle according to any of claims 2 to 4, wherein a first taper part inclined to the inside from the tip end side towards the rear end side is provided on the outer circumferential surface of the needle tube, a second taper part inclined to the inside from the rear end side towards to the tip end side is provided at one end of the ventilation part, and the ventilation part is fixed to the needle tube with an end part of the second taper part abutting the first taper part of the needle tube.

6. The injection needle according to any of claims 2 to 4, wherein a third taper part having a wall thickness that increases towards the outside moving from the tip end side to the rear end side is provided at one end of the ventilation part.

7. The injection needle according to claim 1, wherein the ventilation part is a groove part formed on the outer circumferential surface of the needle tube across the first position and the second position.

8. The injection needle according to claim 1, wherein the ventilation part comprises a groove part formed on the outer circumferential surface of the needle tube across the first position and the second position, and a tube-shaped member provided across a part or the whole of the groove part and forms a gap between the inner circumferential surface and the groove part when the needle tube is inserted internally.

9. The injection needle according to any of claims 1 to 8, further comprising a needle base for retaining the rear end side of the needle tube, wherein the first position and the second position are located nearer to the tip end of the needle tube than the needle base.

10. An injector comprising the injection needle according to any of claims 1 to 9.

11. A co-injection device comprising an injector operation unit capable of operating the injector according to claim 10; and an injection control unit for controlling the injector operation unit and injecting a liquid into a container with the injection needle of the injector puncturing into a rubber stopper of the container up to a position between the first position and the second position.

12. A co-injection device comprising an injector operation unit capable of operating the injector according to claim 10; and a suction control unit for controlling the injector operation unit and sucking a liquid from inside a container with the injection needle of the injector puncturing into a rubber stopper of the container up to a position between the first position and the second position.

13. A co-injection method executed by a co-injection device using the injector according to claim 10, the method comprising a first puncture step for controlling the operation unit and puncturing the rubber stopper of the container with the injection needle of the injector up to a position between the first position and the second position; and an injection step for injecting a liquid into the container with the injection needle puncturing the rubber stopper according to the first puncture step.

14. The co-injection method according to claim 13, further comprising a second puncturing step for controlling the operation unit and puncturing the rubber stopper of the container with the injection needle of the injector up to a position positioned nearer to the tip end than the first position; and an injection step for sucking a liquid from the container with the injection needle puncturing the rubber stopper according to the second puncture step.

15. A co-injection method executed by a co-injection device using the injector according to claim 10, the method comprising a third puncture step for controlling the operation unit and puncturing the rubber stopper of the container with the injection needle of the injector up to a position between the first position and the second position; and a suction step for sucking a liquid from the container with the injection needle puncturing the rubber stopper according to the third puncture step.
